# EUROPEAN PATENT SPECIFICATION

(11) **EP 2 228 015 B1**
(45) Date of publication and mention of the grant of the patent: **14.09.2016**
(21) Application number: 09251495.9
(22) Date of filing: 05.06.2009
(51) Int. Cl.: A61B 17/04, A61B 17/68

(54) **ATTACHMENT DEVICE**
BEFESTIGUNGSVORRICHTUNG
DISPOSITIF DE FIXATION

(30) Priority: 09.03.2009 US 400735
(43) Date of publication of application: 15.09.2010
(73) Proprietor: Stout Medical Group, L.P., Perkasie PA 18944 (US)
(72) Inventor: Greenhalgh, E. Skott, Lower Gwynedd, PA 19002 (US); Keifer, Robert A., Quakertown, PA 18951 (US); Vishnubhotla, Srilakshmi, San Diego, CA 92122 (US)
(74) Representative: Forsythe, Dominic

(56) References cited:
- EP-A1- 1 405 607
- EP-A1- 2 131 767
- EP-A2- 0 673 624
- WO-A1-01/50967
- WO-A2-2007/131002
- WO-A2-2008/004057
- JP-A- 8 299 362
- US-A- 4 013 071
- US-A- 5 472 452
- US-A1- 2005 038 437
- US-A1- 2007 093 899

## Description

### BACKGROUND OF THE INVENTION

### 1. Field of the Invention

The present invention relates to an attachment device for deployment to biological tissue.

### 2. Description of Related Art

Broken bones, such as compression fractures of one or more vertebrae in the spine, may be treated with internal fixation. Any indication needed spinal stability can also be treated by internal fixation. Examples include scoliosis, kyphosis, spondylothisthesis and rotation, segmental instability, such as disc degeneration and fracture caused by disease and trauma and congenital defects, and degeneration caused by tumors.

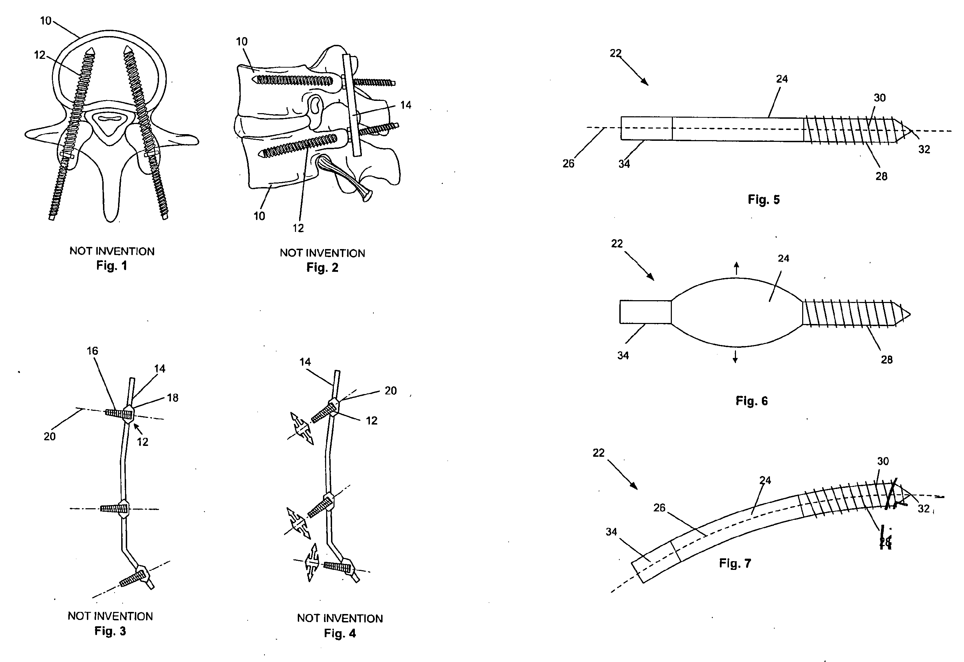

As shown by Figure 1, internal fixation in the spine is often accomplished by first screwing fixation screws into the pedicles and vertebral bodies of the vertebrae 10. Figure 2 shows that the fixation screws are then typically attached to a rigid fixation rod or plate that provide support between one or more weakened vertebra 10. This support often immobilizes the vertebra 10 to which the fixation screws have been inserted.

Figure 3 illustrates that existing fixation systems often have the fixation rod 14 or plate 220, through which a number of fixation screws 12 are deployed. The screw head 18 prevents the fixation rod 14 from separating from the fixation screw 12. The fixation screw 12 also has a screw body 16 which has a screw longitudinal axis 20 often static relative to the fixation rod 14.

Figure 4 illustrates that in some existing fixation systems, the fixation screws 12 can be polyaxial screws: attached to the fixation rod 14 or plate 220 in a manner so that the screw longitudinal axis 20 can rotate, as shown by arrows, with respect to the fixation rod 14.

Backing out or loosening of the fixation screws 12 can cause a reduction of the fixation, up to complete failure or even resulting in additional complications.

Furthermore, the bones are often weak and under heavy loads, the bones can fail and the fixation screws 12 can be ripped from the bone resulting in complete failure and additional damage to the bone.

Therefore, a fixation screw that can substantially eliminate the risk of backout, and can provide a higher anchoring force is desired. A fixation screw that can also minimize bone failure is desired.

EP2131767A1 is considered the prior art closest to the invention as claimed. EP 2 131 767 A1 discloses an expandable attachment having a radially expandable section and a distal end. The distal end can be configured to be attached to a separate device, such as a fixation rod or plate. The device can have an unexpandable section. Also disclosed is an expandable attachment device that can have a radially expandable section and an unexpandable section. The unexpandable section and/or the radially expandable section can have external threads. The devices can be used as substitutes for fixation screws in existing fixation systems. The devices can be used to treat broken bones, scoliosis, kyphosis, spondylolisthesis and rotation, segmental instability, such as disc degeneration and fracture caused by disease and trauma and congenital defects, and degeneration caused by tumors. The devices can be configured to be used in systems with fixed screw longitudinal axis or polyaxial configurations.

US 4 013 071 A discloses fasteners for use in orthopedic surgery. The fasteners are particularly useful as orthopedic screws and comprise an external fastening member having a shank formed with a head at one end, external spiral threads extending to the opposite end, an internal bore extending axially through the head of the shank, and a plurality of side slits formed radially through the tip at the opposite end of the shank to the bore to provide a plurality of outwardly expansible tips at the latter end. The fastener also includes, an internal spreading member axially movable within the bore to engage the tips of the fastening member and thereby to outwardly spread them.

### SUMMARY OF THE INVENTION

According to the present invention there is provided the attachment device of claim 1.

There are hereinafter described and illustrated expandable attachment devices and methods for using the same. An expandable attachment device can have a radially expandable section and a distal end. The distal end can be configured to be attached to a separate device, such as a fixation rod or plate. The device can have an unexpandable section.

Also disclosed is an expandable attachment device that can have a radially expandable section and an unexpandable section. The unexpandable section and/or the radially expandable section can have external threads.

The devices described herein can be used as substitutes for fixation screws in existing fixation systems. The devices can be used to treat broken bones, scoliosis, kyphosis, spondylothisthesis and rotation, segmental instability, such as disc degeneration and fracture caused by disease and trauma and congenital defects, and degeneration caused by tumors.

The devices can be configured to be used in systems with screws with a fixed longitudinal axis or moveable polyaxial axes.

The device can be made from multiple unibody pieces, such as being a two-piece device. The expanding screw can be made from two components, for example, an outer shell and an inner structural element. The outer shell can be made from materials such as medical implant grade metals, polymers, any material disclosed herein, or combinations thereof. For example, the outer shell can be made from a metal with a high ductility (e.g., grade 2 Ti and/or steel).

The inner structural element can support a majority, minority or equal amount of the mechanical load compared with the outer shell during implantation and/or over the life of the use of the device. Loads on the device can include torsion, bending stiffness, hysteresis fatigue, compression, tension, shear, other loads, or combinations thereof. The inner structural element can be made from any one or multiple material disclosed herein, such as a high strength material. For example, the inner structural element can be made from alloy grade Ti 5, high strength steel, or combinations thereof.

The device can be screwed into a bone in the spine and/or other tissue in the body (e.g., femur, tibia). The outer shell and/or inner structural element can have one or more anti-torque elements. The anti-torque elements can increase the resistance to a torque failure of the shell relative to the inner structural element. The outer shell can be cannulated (e.g., have a hollow length). The inner structural element can be cannulated. The inner structural element can be partially or completely inserted into the hollow length of the outer shell.

The anti-torque element can be thread on the inner radius (i.e., the wall of the hollow length) of the outer shell and/or outer radius of the inner structural element. The anti-torque element of the outer shell and/or inner structural element can rotationally attach to the other component (i.e., the inner structural element and/or outer shell, respectively). For example, the anti-torque thread on the inner radius of the outer shell can engage the anti-torque thread on the outer radius of the inner structural element or the smooth or textured wall surface of the outer radius of the inner structural element (e.g., when the outer radius of the inner structural element does not have threads at all or threads that align with the threads on the outer shell).

The anti-torque element can be configured as a sloped wall of the channel forming the hollow length within the outer shell and/or a sloped wall of the outer radius of the inner structural element. The one or both (i.e., on the outer shell and/or inner structural element) sloped walls can form a press-fitting between the outer shell and the internal structural element.

The anti-torque elements can be or have pins in slots, detents in slots, a distal cap laser welded on after the shell is threaded on, or combinations of any of the anti-torque elements disclosed herein. For example, the anti-torque element can be a combination of thread(s) and/or a sloped wall(s) and/or a pin(s) and channel(s).

The anti-torque element (e.g., the inner thread on the outer shell) on a first component (e.g., the outer shell) can engage the second component (e.g., the inner structural element) after the first component contacts or otherwise attaches to the second component.

Any of the anti-torque elements listed above can be used in combination.

A filler can be pumped or otherwise delivered through the cannulated hollow length of the inner structural element and/or the outer shell, and out the terminal distal end of the device. The inner element and/or outer shell can have holes or fenestrations through the inner element walls. The filler can flow out the fenestrations in the side of the device between the proximal and distal ends. The filler can flow through and around the device, expanded screw struts, and into the surrounding tissue (e.g., bone). The filler can press moving or flexible elements of the device together or apart, for example locking the device in place and in the deployed configuration. The holes can be various shapes, sizes, slots, a single hole, or multiple groups of holes or spaces holes. The holes can be under the outer shell strut section, or the holes can align with non stent strut holes in the shell not designed to expand.

The filler can have any materials described herein, for example PMMA, BMP's, calcium sulphate, calcium phosphate, or combinations thereof.

An inner deployment rod can connect the inner element and the outer shell, for example to expand the screw. A tensile (compressive force) load can be applied to the rod pulling the distal end of the shell towards the distal end of the inner element.

The deployment rod can be hollow, having a hollow channel along the length of the deployment rod. The hollow channel in the deployment rod can open through a distal port. The hollow channel can be in fluid communication through fenestrations or holes in the side of the deployment rod. The filler can be inserted under pressure through a proximal port in the hollow channel through the rod and/or the rod can be removed and the filler can then be injected in the hollow length of the inner structural element. The rod can the can be placed back into the internal structural element (or otherwise into the device), for example to reinforce the inner structural element (e.g., making the device stiffer to bending loads). The rod deployment rod can be left in place or removed after longitudinal compression of the device, and/or after deployment of the filler.

The outer shell can be fixed or locked to the inner structural element. The outer shell can be fixed to the inner structural element along the length of the outer shell and/or proximal to the outer shell. The outer shell can be fixed to the inner structural element by threading, press-fitting, welding, thermal shrink fitting, pinning, staking, detenting, or combinations thereof. The fixation can occur at the proximal end of the device via a first method, and at the distal end of the device via a second method. For example, the distal end of the outer shell can be locked to the inner structural element with one or more locking pins and the proximal end of the outer shell can be press-fitted into the proximal end of the inner structural element. The distal and proximal parts of the screw can remain fixed together, for example even if distal shell elements fracture.

The distal end of the inner surface of the outer shell can interference fit against the outer surface of the inner structural element. The interference fit of the outer shell and the inner structural element can occur during radial expansion (e.g., as the inner structural element is pushed or twisted into the outer shell). The interference fit can be designed to limit radial expansion of the device. For example, a longer internal structural element can be used with the same outer shell to result in less radial expansion. Alternatively, a shorter internal structural element can be used with the same outer shell to result in more radial expansion of the device.

The inner element can have a tapered section or shoulder to create a natural pressed fit with the outer shell. The press-fitted inner shell can take most of the load relative to the outer shell when bending loads are applied to the device.

The device can be radially unexpanded (i.e., radially contracted) by removing part or all of the inner structural element from the outer shell. The tensile loads in the outer shell can cause the struts or outer wall to bend back to the original shape

The outer shell can have other expanding elements as disclosed herein, for example ramps, skins, sliding elements or combinations thereof.

Also disclosed is a method of preparing or testing an attachment device ready for use in attaching to biological tissue at a target site, the method comprising:
providing an inner structure;
providing an expandable outer shell having an outer shell hollow length; and
assembling the attachment device by inserting the inner structure completely or partially into the outer shell hollow length;
wherein the assembled attachment device is constructed and arranged to be deliverable to the target site to enable the outer shell to be expanded in the target site and filler to be delivered to the target site through the inner structure and the outer shell. The preparation or testing method is an *ex-vivo* method that does not require the intended patient to be present and thus is not a method for treatment of the human or animal body by surgery or therapy or a diagnostic method practised on the human or animal body, within the meaning of Article 53(c) EPC.

### BRIEF SUMMARY OF THE DRAWINGS

Figure 1 is a partially see-through top view of a vertebra with fixation screws therethrough.
Figure 2 is a partially see-through lateral view of a section of the spine with fixation screws and a fixation rod.
Figures 3 and 4 illustrate simplified variations of existing fixation systems.
Figure 5 illustrates a variation of the expandable attachment device in a radially contracted configuration.
Figure 6 illustrates the variation of the expandable attachment device in a radially expanded configuration.
Figure 7 illustrates a variation of the expandable attachment device in a radially contracted configuration.
Figures 8 and 9 illustrate a variation of the expandable attachment device and a method for radially expanding the device.
Figures 10 and 11 illustrate a variation of the expandable attachment device and a method for radially expanding the device.
Figures 12 and 13 illustrate a variation of the expandable attachment device and a method for radially expanding the device.
Figures 14 and 15 illustrate a variation of the expandable attachment device and a method for radially expanding the device.
Figures 16 and 17 illustrate a variation of the expandable attachment device and a method for radially expanding the device.
Figure 18 illustrates a variation of the expandable attachment device in a contracted configuration.
Figures 19 and 20 illustrate variations of the expandable attachment device of Figure 18 and methods for radially expanding the device.
Figure 21 illustrates a variation of the expandable section in a radially contracted configuration.
Figure 22 illustrates the expandable section of Figure 21 in a radially expanded configuration.
Figure 23 illustrates a variation of the expandable section in a radially contracted configuration on the expandable attachment device.
Figure 24 illustrates a variation of the expandable section in a radially expanded configuration on the expandable attachment device.
Figures 25a through Figure 25e illustrate variations of the expandable section.
Figures 26 and 27 illustrate a variation of the expandable attachment device and a method for radially expanding the device.
Figures 28 and 29 illustrate a variation of the expandable attachment device and a method for radially expanding the device.
Figures 30 and 31 illustrate variations of the expandable attachment device.
Figures 32 and 33 are side and end perspective views, respectively, of a variation of the expandable attachment device.
Figure 34 is a side view of a variation of the expandable attachment device.
Figures 35a and 35b illustrate a variation of the expandable section.
Figure 36 is a side view of the expandable section of Figures 35a and 35b.
Figure 37 is a variation of a close-up view of section A-A of Figure 36.
Figure 38 is a flattened view of a variation of the expandable section.
Figure 39 is a variation of a close-up view of section B-B of Figure 38.
Figures 40a and 40b are flattened views of variations of the expandable section.
Figure 41 illustrates a variation of the unexpandable section integral with the central shaft and distal end of the expandable attachment device.
Figure 42 illustrates a variation of cross-section C-C of Figure 41.
Figure 43 illustrates a variation of cross-section D-D of Figure 41.
Figure 44 is a variation of a close-up E-E of Figure 42.
Figure 45 is a distal end view of a variation of the unexpandable section integral with the central shaft and distal end of the expandable attachment device of Figure 41.
Figure 46 illustrates a variation of the center shaft integral with the unexpandable section and the distal end.
Figures 47a and 47b are various perspective views of a variation of the proximal end cap.
Figure 48 is a side view of a variation of the proximal end cap.
Figure 49 is a distal end view of a variation of the proximal end cap.
Figure 50 illustrates a variation of cross-section Z-Z of Figure 47a.
Figure 51 illustrates a variation of cross-section Y-Y of Figure 47b.
Figure 52 illustrates a variation of the expandable attachment device attached to a variation of the deployment tool.
Figures 53 and 54 illustrate a variation of the expandable attachment device in unassembled and assemble configurations, respectively, and a method for assembling the expandable attachment device.
Figure 55 illustrates a variation of the deployment tool in an unassembled configuration.
Figure 56 is a close-up perspective view of the end of the deployment tool in an assembled configuration.
Figure 57 illustrates a variation of the expandable attachment device in radially expanded configurations, and measurements thereof. Figures 58 and 59 illustrate a variation of the expandable attachment device and a method for radially expanding the device.
Figures 60 and 61 illustrate a variation of the expandable attachment device and a method for radially expanding the device.
Figure 62 illustrates a variation of the expandable attachment device and a method for radially expanding the device.
Figures 63 and 64 illustrate a variation of the expandable attachment device and a method for radially expanding the device.
Figure 65 illustrates a variation of cross-section F-F of Figure 64.
Figure 66 is a perspective view of a variation of the expandable section in a radially contracted configuration.
Figure 67 is an end view of the variation of the expandable section of Figure 66 in a radially contracted configuration.
Figure 68 is an end view of the variation of the expandable section of Figure 66 in a radially expanded configuration.
Figures 69 and 70 are perspective views of variations of the expandable section.
Figure 71 illustrates a variation of the expandable section with the deployment rod.
Figures 72 and 73 illustrate variations of cross-section W-W of Figure 71.
Figures 74 and 75 illustrate variations of cross-section W-W of Figure 72.
Figures 76 and 77 illustrate a variation of the expandable section of Figure 70 with a wedge, and a method for using the same.
Figure 78 illustrates a variation of cross-section V-V of Figure 77.
Figures 79a, 79b, 79c, and 79d illustrate perspective, top, side, and rear views of a variation of the manipulation tool.
Figures 80 through 82 illustrate a variation of the expandable section and a method for radially expanding the same.
Figures 83 and 84 illustrate variations of the expandable section.
Figures 85 and 86 illustrate various perspective views of a variation of the expandable attachment device in a radially contracted configuration.
Figure 87 illustrates a variation of cross-section G-G of Figure 86.
Figures 88 and 89 illustrate various perspective views of the variation of the expandable attachment device of Figures 85 through 87 in a radially expanded configuration.
Figures 90 and 91 illustrate a variation of the expandable attachment device and a method for radially expanding the device.
Figures 92 and 93 illustrate a variation of the expandable attachment device and a method for radially expanding the device.
Figure 94 illustrates a variation of the expandable attachment device and a method for radially expanding the device.
Figures 95 and 96 illustrate proximal end views of variations of the expandable attachment device.
Figures 97 and 98 illustrate a variation of the expandable section in radially contracted and expanded configurations, respectively.
Figures 99 and 100 are side and proximal end views, respectively, of a variation of the expandable section with the center shaft.
Figures 101 and 102 are side and proximal end views, respectively, of a variation of the expandable section.
Figures 103 and 104 are front and side perspective views, respectively, of a variation of the expandable element.
Figures 105 through 107 illustrate variations of the expandable element.
Figures 108 and 109 illustrate a variation of the expandable section and distal end and a method for radially expanding the device.
Figures 110 and 111 illustrate variations of the expandable section.
Figures 112 and 113 illustrate a variation of the expandable attachment device and a method for radially expanding the device.
Figure 114 illustrates a variation of the expandable element of Figures 112 and 113.
Figure 115 illustrates a variation of cross-section K-K of Figure 114.
Figures 116 and 117 illustrate cross-sections H-H and J-J, respectively, of Figures 112 and 113, respectively.
Figures 118 and 119 illustrate a variation of the expandable attachment device and a method for radially expanding the device.
Figure 120a illustrates a variation of multiple expandable elements.
Figure 120b is an end view of a variation of the expandable section in a contracted configuration.
Figure 120c is an end view of a variation of the expandable section in a radially expanded configuration and a method for radially expanding the expandable section.
Figures 121, 122, 123 and 124 are side, perspective, distal end, and proximal end views, respectively, of a variation of the expandable attachment device in a radially contracted configuration.
Figures 125, 126, and 127 are distal end, proximal end, and side views, respectively, of a variation of the expandable attachment device of Figures 121 through 124 in a radially expanded configuration.
Figures 128 and 129 are front and perspective views, respectively, of a variation of the expandable section in a radially contracted configuration.
Figures 130 and 131 are front and perspective views, respectively, of the variation of the expandable section of Figures 128 and 129 in a radially expanded configuration.
Figures 132 and 133 are front and perspective views, respectively, of the variation of the expandable section of Figures 128 and 129 in a radially expanded configuration.
Figures 134 and 135 are perspective and side views, respectively, of a variation of the center shaft.
Figure 136 is an end view of a variation of the expandable section in a radially contracted configuration.
Figure 137 is an end view of the expandable section of Figure 136 in a radially expanded configuration.
Figure 138 is a perspective view of the first expandable element and the second expandable element of Figure 137.
Figure 139 is a perspective view of the expandable section of Figure 137.
Figures 140 through 142 illustrate variations of the expandable section in radially contracted configurations.
Figure 143 illustrates a variation of the expandable attachment device with the expandable section of Figure 141.
Figure 144 illustrates an unassembled expandable attachment device of Figure 143.
Figure 145 illustrates a variation of cross-section L-L of Figure 143 during use.
Figure 146 illustrates a variation of the expandable attachment device.
Figures 147, 148 and 149 illustrate variations of the expandable attachment device with the expandable section of Figures 140, 141 and 142, respectively.
Figures 150 and 151 illustrate side and perspective views, respectively, of a variation of the expandable section in a radially contracted configuration.
Figures 152 and 153 illustrate variations of the expandable section in radially expanded configurations.
Figures 154a and 154b are side and see-through line side views, respectively, of a variation of the attachment device.
Figures 154c and 154d are side and see-through line side views, respectively, of a variation of the attachment device.
Figures 154e through 154j are variations of cross-section Q-Q of Figure 154d.
Figure 154k is a side view of a variation of the attachment device.
Figure 154l is a see-through line side views of a variation of the attachment device.
Figure 155a illustrates a method for inserting a variation of the inner structure into a variation of the outer shell.
Figure 155b illustrates a see-through view of a method for inserting a variation of the inner structure into a variation of the outer shell.
Figures 156a and 156b are side and see-through line side views, respectively, of a variation of the outer shell.
Figures 157a and 157b are side and see-through line side views, respectively, of a variation of the inner structure.
Figures 158a and 158b are side and see-through line side views, respectively, of a variation of the outer shell.
Figures 158c through 158f are variations of cross-section X-X of Figure 158a.
Figures 159a and 159b are side and see-through line side views, respectively, of a variation of the inner structure.
Figures 159c through 159g are variations of cross-section R-R of Figure 159a.
Figures 160a and 160b are side and partial see-through side views, respectively, of a variation of the attachment device.
Figures 160c through 160f are variations of cross-section XR-XR of Figure 160a.
Figures 161 is a variation of close-up section N-N of Figure 160b.
Figures 162a and 162b are variations of close-up section P-P of Figure 160b.
Figure 163 is a lateral view of the spine.
Figure 164 illustrates cross-section M-M of Figure 163.
Figure 165 illustrates cross-section M-M of Figure 163 with an expandable attachment device delivered into the pedicle and/or vertebral body.
Figure 166 is a partial see-through lateral view of the spine with a variation of the expandable attachment device delivered to, and radially expanded in, the pedicle and/or vertebral body.
Figure 167 illustrates cross-section M-M of Figure 166.
Figure 168 illustrates a variation of a method for using a variation of the expandable attachment device to treat a broken bone.
Figure 169 illustrates a variation of a method for using two variations of the expandable attachment devices to treat a broken bone.
Figures 170 and 171 illustrate a variation of a method for attaching an end attachment to the remainder of a variation of the expandable attachment device.
Figure 172 illustrates a variation of method for using a variation of the expandable attachment devices with a fixation rod in the spine.
Figure 173 illustrates a variation of a method for using a variation of the expandable attachment devices with end attachments in the spine.
Figures 174 through 176 illustrate a variation of a method for expanding first and second expandable sections on a variation of the expandable attachment device.
Figures 177 and 178 illustrate variations of methods for using a variation of the expandable support device in the spine.
Figure 179 is an anterior view of a variation of a method for using the expandable attachment device in a spine with a fixation plate.
Figures 180 and 181 are sagittal cross-sections of a variation of a method for using the expandable attachment device in a spine with a fixation plate.
Figure 182 illustrates a variation of the deployment tool.
Figures 183 through 187 illustrate a variation of a method for implanting a variation of the expandable attachment device for use as a tooth anchor.
Figures 188 and 189 illustrate a variation of a method for implanting a variation of the expandable attachment device for use as a tooth anchor.
Figure 190 illustrates a variation of the expandable attachment device.
Figure 191 is a close-up view of the expandable attachment device of Figure 190.
Figure 192 illustrates cross-section S-S of the expandable attachment device of Figure 190.
Figure 193 illustrates a variation of close-up section T-T of the expandable attachment device of Figure 192.
Figure 194 is a close-up view of a variation of the expandable attachment device.
Figure 195 is an expanded view of the expandable attachment device of Figure 194.
Figure 196 illustrates a variation of cross-section U-U of Figure 195.

### DETAILED DESCRIPTION

Figure 5 illustrates that the expandable attachment device 22 can have an unexpandable section 28 at a proximal end, an expandable section 24 at a medial length along the expandable attachment device 22, and a proximal end 34. In other variations of the expandable attachment device, 22 the unexpandable section 28 can be distal to the expandable section 24, and/or the expandable attachment device 22 can have more than one expandable section 24 and/or unexpandable section 28 that can be interspersed with each other.

The expandable attachment device 22 can have an expandable attachment device 22 axis. The expandable device axis 26 can be substantially straight or curved.

The proximal end of the expandable attachment device 22 can have a tip 32. The tip 32 can be sharpened or otherwise configured to seat the expandable attachment device 22 in bone 228 (e.g., having cutting teeth). The unexpandable section 28 can have unexpandable thread 30, for example, configured to screw the expandable attachment device 22 into bone 228.

Figure 5 illustrates that the expandable attachment device 22 can have a radially contracted configuration. Figure 6 illustrates that the expandable attachment device 22 can have a radially expanded configuration. For example, the expandable section 24 can be radially expanded, as shown by arrows.

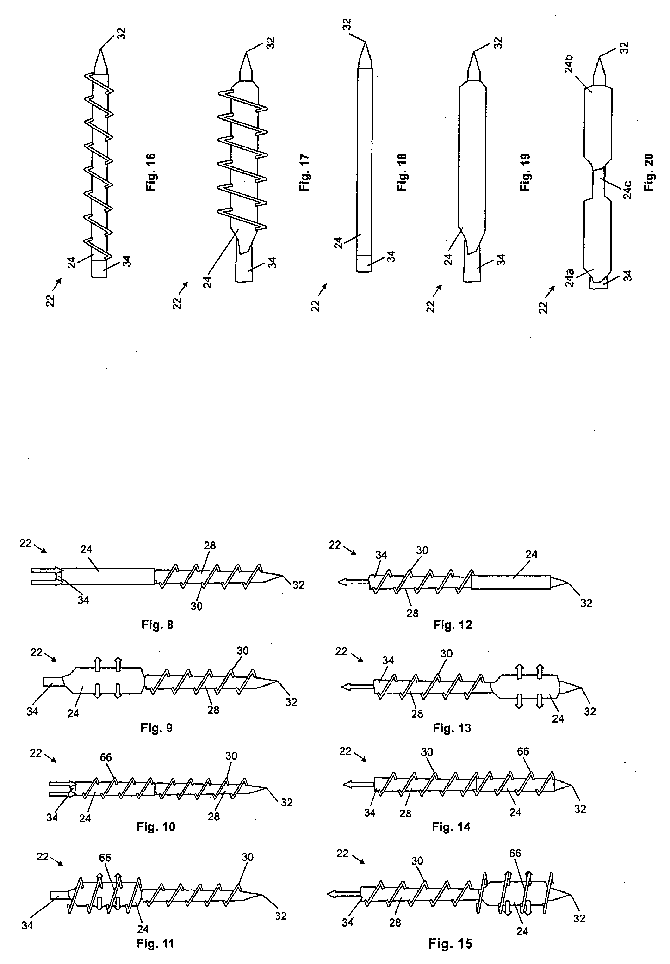

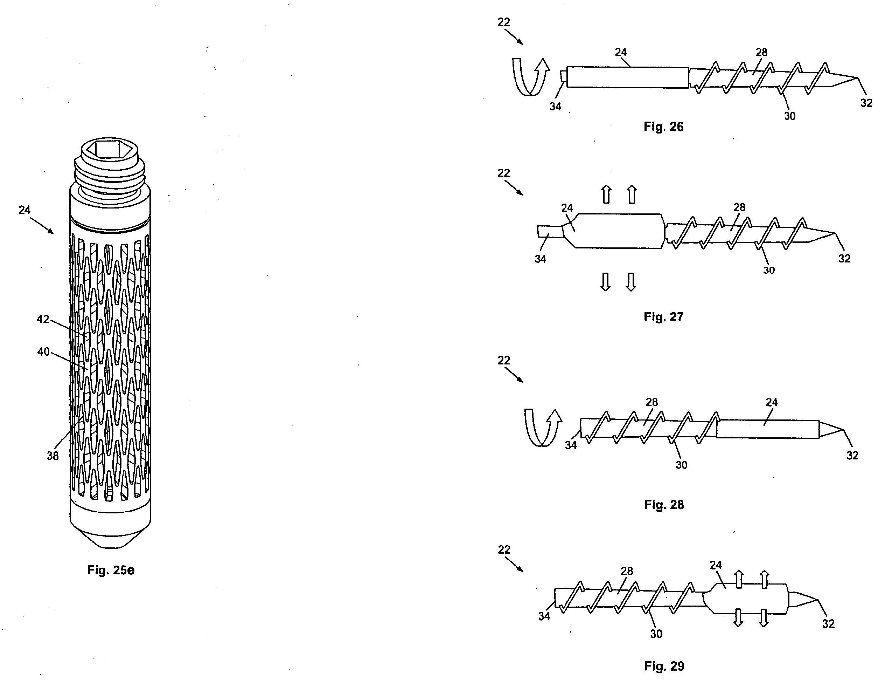

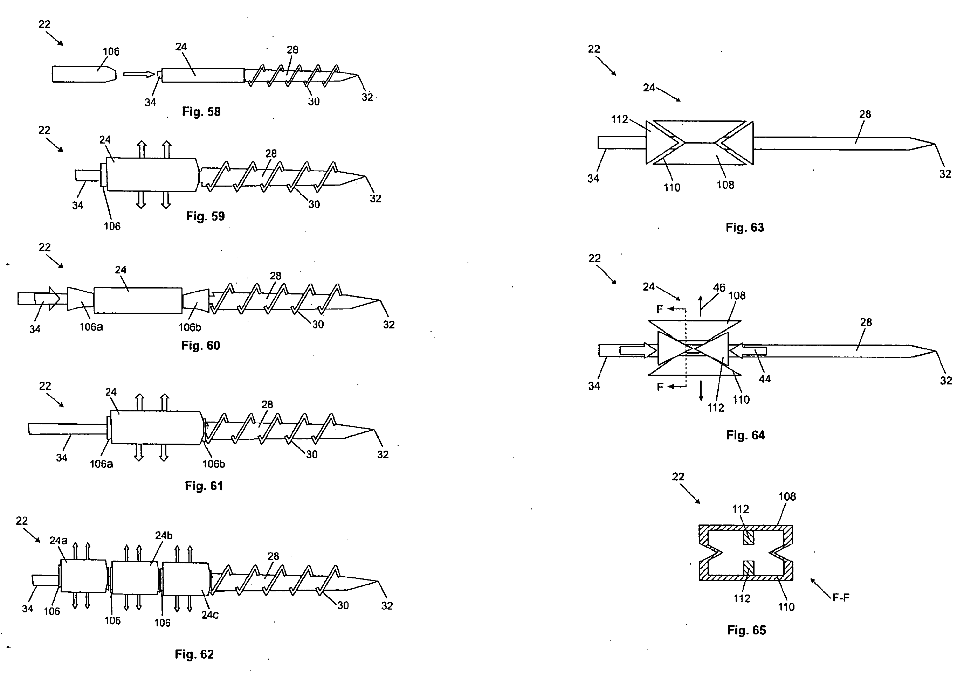

The expandable section 24 can be resiliently and/or deformably expandable. The expandable sections 24 can be radially expanded by axial compression (e.g., see Figs. 8-11), rotation (e.g., see Figs. 26-29), use of a lever such as a wedge 130, ramp 110 or jack (e.g., see Figs. 58-64), or combinations thereof.

The expandable section 24 can be biased to resiliently radially expand. For example, the expandable section 24 can be self-expandable or releasable spring. The expandable section 24 can be resiliently radially expandable and can be additionally deformably radially expandable to a larger radius than achieved by resilient expansion alone.

The expandable section 24 can have one or more anchors extending radially therefrom when the expandable section 24 is in the radially expanded configuration. The anchors can be brads, hooks, pins, teeth, fasteners, pegs 152, screws, skewers, spikes, stakes, or combinations thereof.

Figure 7 illustrates that the expandable attachment device 22 axis can be curved. The expandable attachment device 22 axis can have curved and straight lengths. For example, the expandable attachment device 22 axis can have a substantially straight length along the unexpandable section 28 and the proximal end 34, and a curved length along the expandable section 24.

Figures 8 and 9 illustrates that the expandable attachment device 22 can be radially expanded by applying a proximally-directed force 132 to the proximal end 34 as shown by arrows of Figure 8. The proximally-directed force 132 can be substantially parallel to the expandable attachment device 22 axis. The proximal force 132 can be opposed by a distal force 132 applied, for example, by the bone 228 and/or a deployment tool 60. The expandable section 24 can then radially expand, as shown by arrows in Figure 9.

Figures 10 and 11 illustrate that the expandable attachment device 22 can have expandable thread 66 on the expandable section 24 and unexpandable thread 30 on the unexpandable section 28. The expandable thread 66 can radially expand with the remainder of the expandable section 24. The expandable attachment device 22 shown in Figures 10 and 11 can be radially expanded by the method as shown in Figures 8 and 9.

Figures 12 and 13 illustrate that the expandable attachment device 22 can be radially expanded by applying a distally-directed force 132 to the proximal end 34 as shown by arrow. The distally-directed force 132 can be substantially parallel to the expandable attachment device 22 axis. The distal force 132 can be opposed by a proximal force 132 applied, for example, by the bone 228 and/or a deployment tool 60. The expandable section 24 can then radially expand, as shown by arrows in Figure 13.

Figures 14 and 15 illustrate that the expandable attachment device 22 can have expandable thread 66 on the expandable section 24 and unexpandable thread 30 on the unexpandable section 28. The expandable thread 66 can radially expand with the remainder of the expandable section 24. The expandable attachment device 22 shown in Figures 14 and 15 can be radially expanded by the method as shown in Figures 12 and 13.

Figures 16 illustrate that substantially the entire length of the expandable attachment device 22 can be the expandable section 24. The proximal end 34 can extend distally from the expandable section 24. Figure 17 illustrates that the entire expandable section 24 can radially expand. Figures 16 and 17 illustrate that the expandable section 24 can have expandable thread 66. Figures 18 and 19 illustrate the variation of the expandable attachment device 22 of Figures 16 and 17, respectively, without expandable thread 66.

Figure 20 illustrates that the expandable attachment device 22 can have, from distal to proximal, a first expandable section 24a, a third expandable section 24c, and a second expandable section 24b. The first, second and third expandable sections 24 can radially expand at different rates (e.g., under different deployment loads, for example one or more are resiliently and one or more are deformably expandable). For example, the first and second expandable sections 24a, 24b can radially expand at the same rate, and the third expandable section 24c can radially expand at a lesser rate.

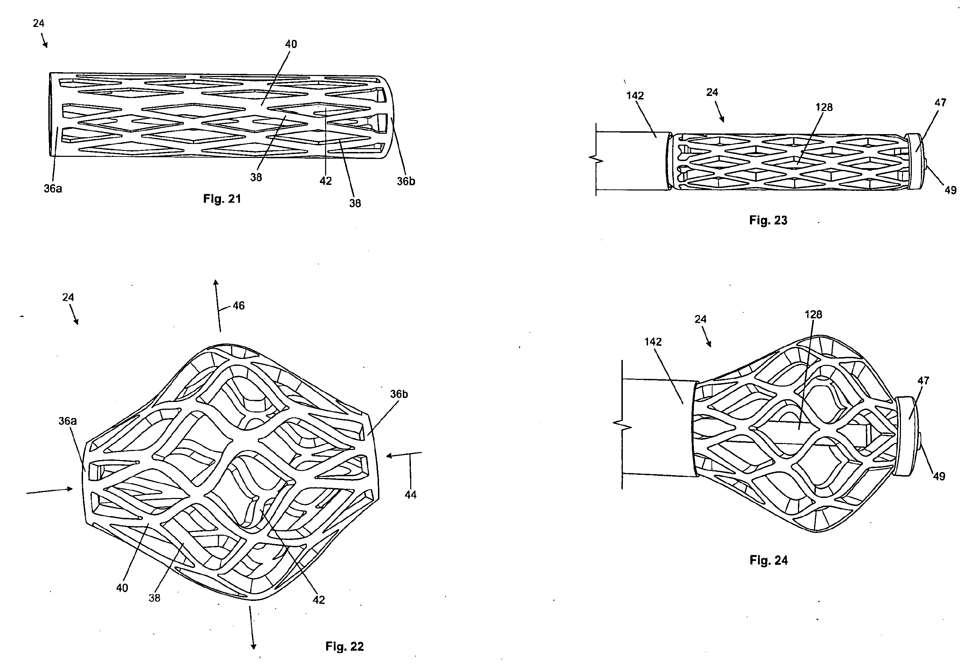

Figure 21 illustrates that the expandable section 24 can have a number of struts 38 attached to each other at joints 40. When the expandable section 24 is in a radially contracted configuration, the struts 38 can be configured to form diamond-shaped ports 42. The expandable section 24 can have a distal hoop 36a at the proximal end 34 and/or a proximal hoop 36b at the proximal end. The hoops 36 can attach to all of the struts 38 at the respective end. The hoops 36 and struts 38 can all be integral with and/or attached to each other.

Figure 22 illustrates that longitudinal compressive force 44 can be applied to the expandable section 24, for example resulting in radial expansion 46. In a radially expanded configuration, the struts 38 can deform near the joints 40. The hoops 36 can remain substantially static.

Figures 23 and 24 illustrates that the expandable section 24 can be radially expanded by longitudinally compressing the expandable section 24. For example, the deployment tool 60 (or expandable attachment device 22) can have an anvil 142 and a deployment cap 47. The anvil 142 can be the proximal end 34 and/or the unexpandable section 28. The deployment cap 47 can be part of or attached to the unexpandable section 28 and/or the proximal end 34, for example, the opposite of the anvil 142. The expandable section 24 can be compressed between the anvil 142 and the deployment cap 47.

The deployment tool 60 (or expandable attachment device 22) can have a deployment rod 128, for example to transmit the compressive force 132 to the deployment cap 47. The deployment rod 128 can be releasably attached to the deployment cap 47, for example via a releasable deployment anchor 49. The releasable deployment anchor 49 can be released and the deployment rod 128 can be removed after the expandable section 24 is radially expanded.

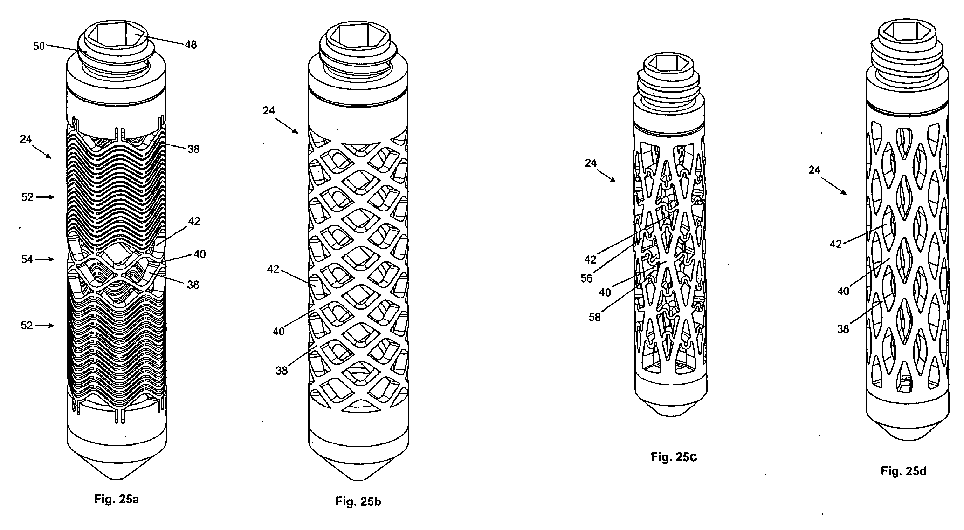

Figures 25a-e illustrate variations of the expandable section's 24 strut 38, port 42 and joint 40 configuration. Figure 25a illustrates that the ports 42 can be larger near a central region 54 near the longitudinal median of the expandable section 24 than in end regions 52. The lengths of the expandable section 24 with larger ports 42 can radially expand during longitudinal compression 44 before the lengths of the expandable section 24 with smaller ports 42. The expandable section 24 can have thread 50 and/or another releasable attachment configuration at one or both ends. The expandable section 24 can have a tool port 48 configured to receive a deployment tool 60 (e.g., a deployment rod 128) through the proximal end of the expandable section 24.

Figure 25b illustrates that the struts 38 and ports 42 can be substantially identical along the entire length of the expandable section 24. Figure 25c can have main struts 56 and smaller folded cross-struts 58 that attach to multiple main struts 56. Figure 25d illustrates that the struts 38 and ports 42 can be substantially identical along the entire length of the expandable section 24 and that the ports 42 can be longer in the longitudinal direction that in the angular direction, with respect to the expandable section 24. Figure 25e that the struts 38 and ports 42 can be substantially identical along the entire length of the expandable section 24 and that the ports 42 can be longer in the longitudinal direction that in the angular direction, with respect to the expandable section 24, and smaller and more numerous than as shown in Figure 25d.

Figures 26 and 27 illustrate that when the proximal end 34 and/or expandable section 24 is rotated, as shown by arrow in Figure 26, that the expandable section 24 can radially expand, as shown by arrows in Figure 27. Figures 26 and 27 illustrate that the expandable section 24 can be distal to the unexpandable section 28.

Figures 28 and 29 illustrate that when the proximal end 34 and/or expandable section 24 is rotated, as shown by arrow in Figure 28, that the expandable section 24 can radially expand, as shown by arrows in Figure 29. Figures 28 and 29 illustrate that the unexpandable section 28 can be distal to the expandable section 24.

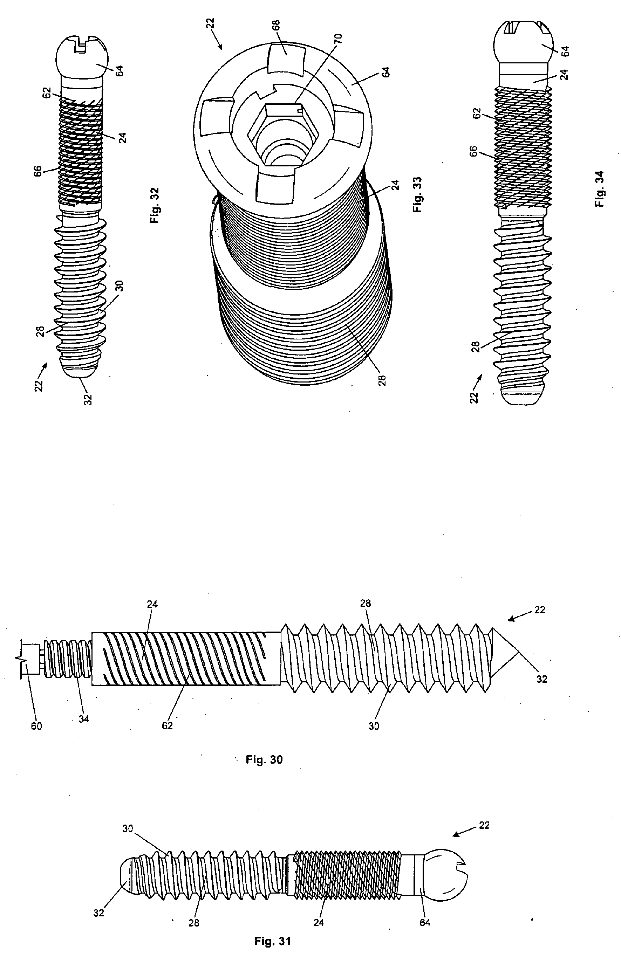

Figure 30 illustrates that the expandable section 24 can have a slot 62 radially through the expandable section 24. The slot 62 can have a helical configuration along the expandable section 24. The proximal end 34 can be threaded 50. The expandable attachment device 22 can be detachably attached to a deployment tool 60.

Figure 31 illustrates that the expandable section 24 can have a textured surface. The expandable attachment device 22 can have a proximal end cap 64 at the proximal end. The proximal end cap 64 can have a substantially spherical configuration.

Figure 32 illustrates that the expandable section 24 can have a helical slot 62 and an expandable thread 66. The expandable thread 66 can be helical at substantially the opposite angle of the helical slot 62. The expandable thread 66 can be helical at a positive or negative angle with respect to a plane perpendicular to the expandable attachment device 22 axis. The helical slot 62 can be helical at the opposite-signed (i.e., positive or negative) angle to the expandable thread 66.

Figure 32 illustrates that the proximal end of the proximal end cap 64 can have cap deployment tool attachments 68, for example cross-notches on the head of the cap 64. The cross-notches can be utilized to engage the proximal end cap 64 with an engagement tool.

The proximal end 34 of the center shaft 80 can have a shaft deployment tool attachment 70, for example, an allen or hexagonal or septagonal socket.

Figure 34 illustrates that when the expandable section 24 is in a radially contracted configuration, the expandable thread 66 can protrude to about the same radius at the unexpandable thread 30 with respect to the expandable attachment device 22 axis.

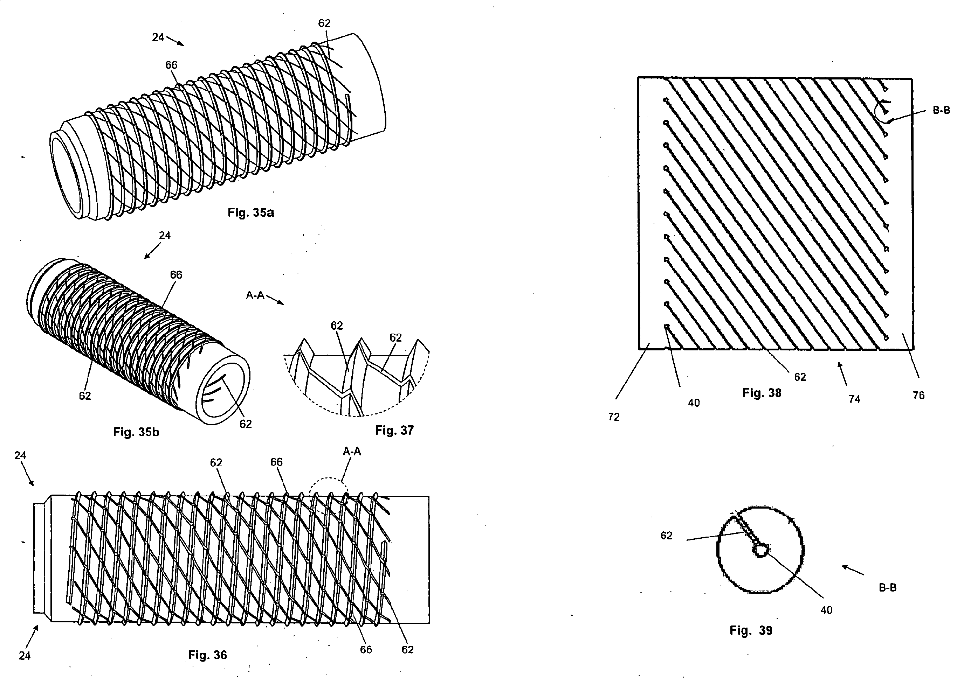

Figures 35a and 35 illustrate that the expandable section 24 can be separate to the remainder of the expandable attachment device 22. Figure 35b illustrates that the helical slot 62 can extend through the thickness of the wall 184 of the expandable section 24. Figures 36 through 39 illustrate additional details of the expandable section 24.

Figure 38 illustrates that the expandable section 24 can have an expandable section wall 72 can have numerous helical slots 62 in a slotted wall section 74. The expandable section wall 72 can have one or more unslotted wall sections 76, for example at the distal 34 and proximal ends of the expandable section 24. The slots 62 can have joints 40 at one both ends of the slots 62.

Figure 39 illustrates that the joints 40 can be circular. The joints 40 can have a larger, smaller or equal diameter to the width of the slot 62.

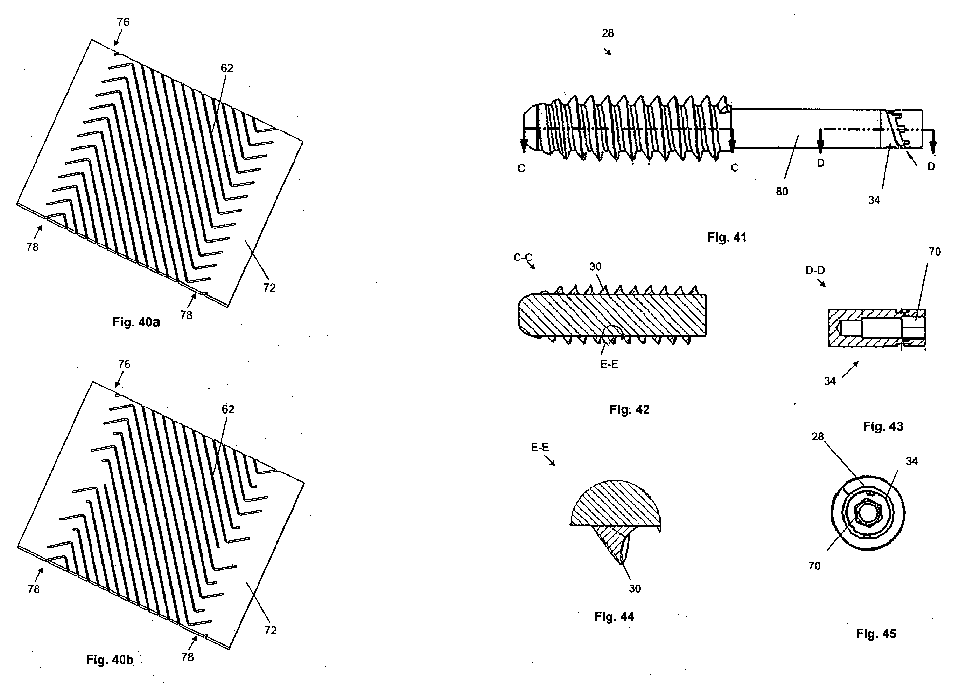

Figure 40 illustrates that the expandable section wall 72 can have one or more retrograde slot sections 78, for example at each end of the slotted wall section 74. The retrograde slot section 78 can have slots 62 in the substantially opposite direction of the slots 62 in the remainder of the slotted wall section 74. The primary (i.e., non-retrograde) slots 62 can be helical at a positive or negative angle with respect to a plane perpendicular to the expandable attachment device 22 axis. The retrograde slots can be helical at the opposite-signed (i.e., positive or negative) angle to the primary slots 62.

The retrograde slot section 78 can, for example, act as a shock absorber. The retrograde slot section 78 can increase maximum radial expansion 46 of the expandable section 24. The slots 62 can be sinusoidal along the length of the expandable section 24.

Figure 40b illustrates that the ends of the slots 62 can be placed at different lengths from the ends of the expandable section 24. For example, varying the lengths of adjacent slots 62 can diffuse strain on the expandable section 24.

Figures 41 through 45 illustrate dimensions of the expandable section 24 (dimensions are shown on attachment B).

Figure 41 illustrates that the unexpandable section 28 can be integral with a center shaft 80 and the proximal end 34.

Figure 43 illustrates that the proximal end 34 can have the shaft deployment tool attachment 70 therethrough.

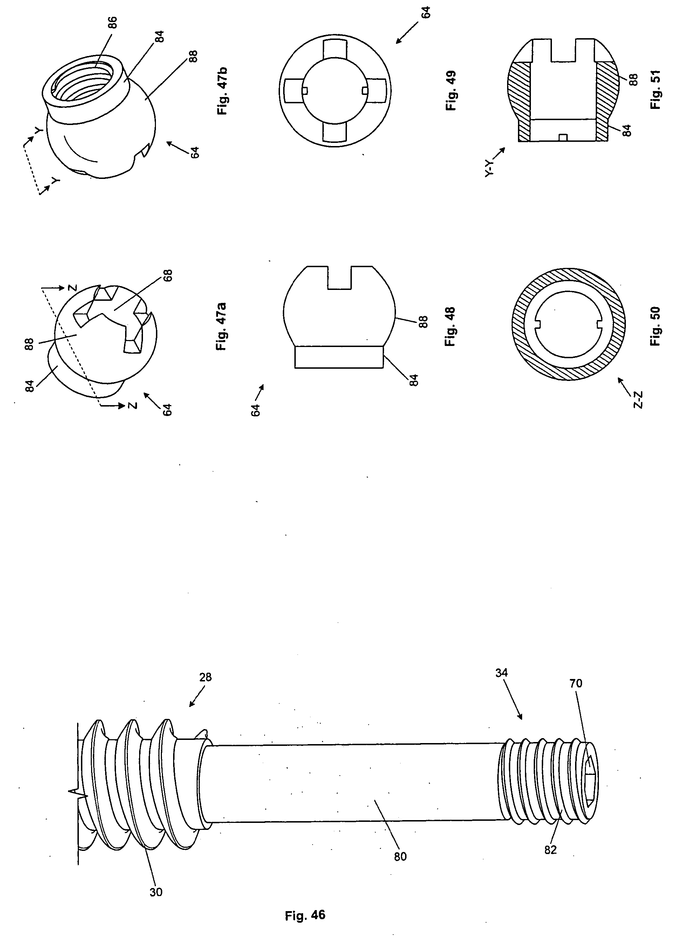

Figure 46 illustrates a close up of the proximal end 34 of the unexpandable section 28, center shaft 80 and proximal end 34.

Figures 47a and 47b illustrate that the distal cap end 64 can have a cap ball 88 and a cap sleeve 84. The cap ball 88 and/or cap sleeve 84 can have internal cap thread 86 along all or part of the length.

Figures 48 through 51 illustrate dimensions of the expandable section 24 (dimensions are shown on attachment C).

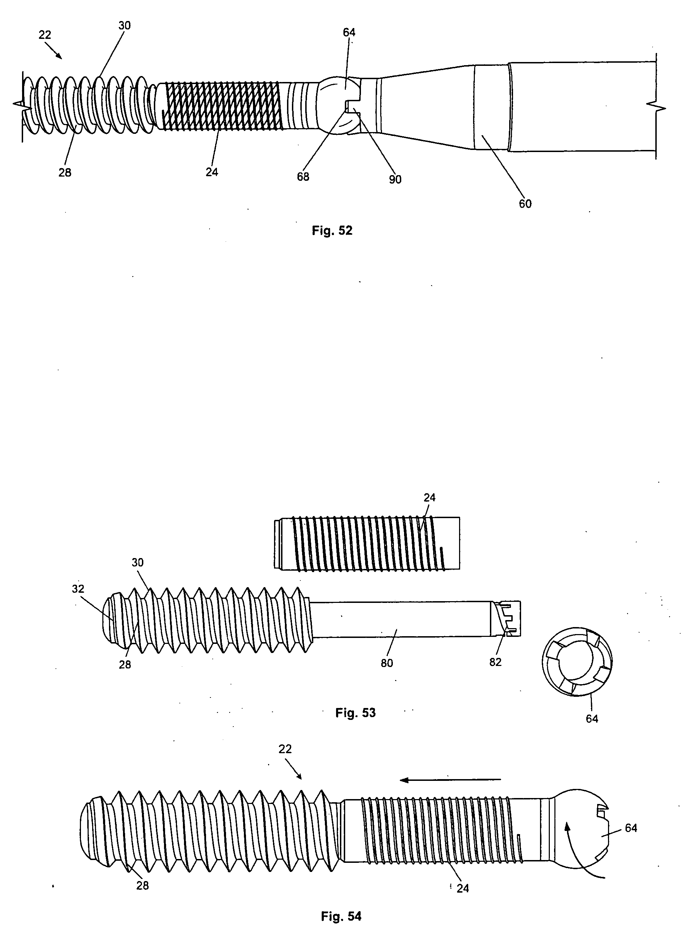

Figure 52 illustrates that the expandable attachment device 22 can be releasably attached to the deployment tool 60. The deployment tool 60 can have deployment engagement teeth 90 that can align and intersect with the proximal end cap 64, for example at the cap deployment tool attachments 68.

Figure 53 illustrates that the expandable attachment device 22 can be dissembled in separate elements. For example, the unexpandable section 28 can be integral with the center shaft 80. The center shaft 80, for example at the proximal end 34, can have shaft cap attachments 82 that can attach to the proximal end cap 64.

Figure 54 illustrates that the expandable attachment device 22 can be assembled by translating the expandable section 24 over the center shaft 80, as shown by arrow. The proximal end cap 64 can then be rotated, as shown by arrow, onto the shaft cap attachments 82.

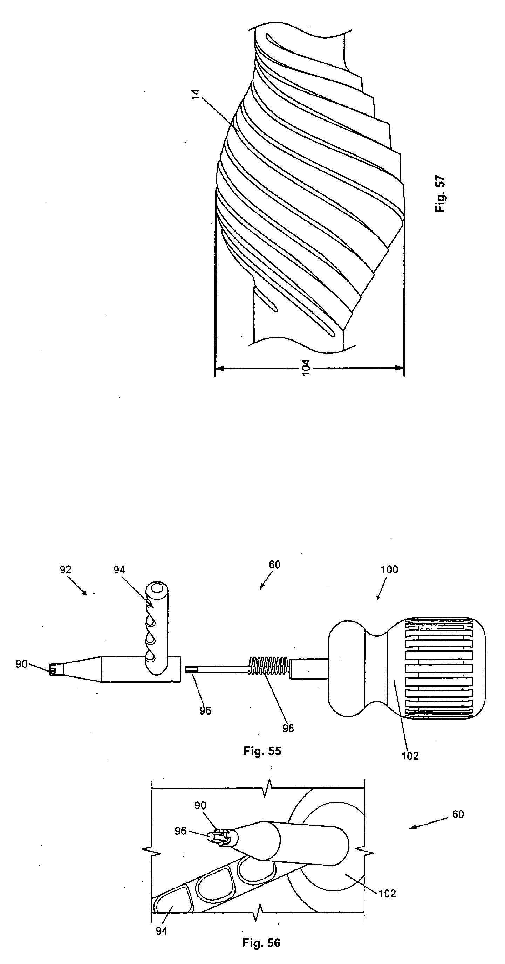

Figures 55 and 56 illustrate that the deployment tool 60 can have a post tool 100 and a tooth tool 92. The tooth tool 92 can be separate, attached, or integral with the post tool 100.

The post tool 100 can have a post tool hand 102. The post tool handle 102 can be attached to or integral with a deployment engagement post 96. The post tool 100 can have a deployment tool suspension 98. The deployment engagement post 96 can be configured to attach to the shaft deployment tool attachment 70.

The tooth tool 92 can have deployment engagement teeth 90. The deployment engagement teeth 90 can be configured to attach to the cap deployment tool attachment 68. The tooth tool 92 can have a tooth tool handle 94, for example extending radially from the remainder of the tooth tool 92.

The deployment tool suspension 98 can resiliently separate the tooth tool 92 and the post tool 100. The deployment tool suspension 98 can suspend the deployment engagement post 96 from the post tool handle 102.

Figure 57 illustrates the expandable section 24 in a radially expanded configuration can have an outer diameter 104 from about 7 mm (0.3 in.) to about 15 mm (0.59 in.), for example about 9.99 mm (0.393 in.) or about 9.31 mm (0.367 in.).

Figures 58 and 59 illustrate that an external wedge 106 can be inserted, as shown by arrow in Figure 58, into the expandable section 24. The expandable section 24 can then radially expand, as shown by arrows in Figure 59. The external wedge 106 can be left in the expandable section 24 or removed from the expandable section 24. The wedge 130 can have a transverse cross section that is square, round (e.g., a conical wedge), rectangular, oval, or combinations thereof.

Figure 60 illustrates that the expandable attachment device 22 can have a first external wedge 106a and a second external wedge 106b. The second external wedge 106b can be attached to or integral with the unexpanded section 28 and/or otherwise positioned between the expandable section 24 and the unexpanded section 28 when the expandable section 24 is in a radially contracted configuration. The second external wedge 106b can be pointing narrow end-first toward the proximal end 34 of the expandable attachment device 22.

A proximally-directed force can be applied, as shown by arrow, to the first external wedge 106a and/or the proximal end 34. The expandable section 24 can then radially expand, as shown by arrows in Figure 61, as the wedges 130 are pushed into a channel in the expandable section 24.

Figure 62 illustrates that the expandable attachment device 22 can have a first expandable section 24a, second expandable section 24b, and third expandable section 24c. The expandable sections 24 can each have one or two external wedges 106 entering into an inner hollow or channel, as shown in Figures 58 through 61.

Figure 63 illustrates that the expandable section 24 can have one or more expansion elements 108 configured to radially expand. The expandable section 24 can have one, two or more internal wedges 112. The expansion elements 108 can have ramps 110 configured to slidably engage the internal wedge 112 when the internal wedge 112 is compressed into the expansion elements 108.

Figure 64 illustrates that the internal wedges 112 can be compressed, as shown by arrows, into the expansion elements 108. The expansion elements 108 can then radially expand, as shown by arrows.

Figure 65 illustrates that the internal wedges 112 can interference fit with the ramps 110. As the internal wedges 112 are further compressed, the internal wedges 112 can cause a deformation or other translation of the expansion elements 108.

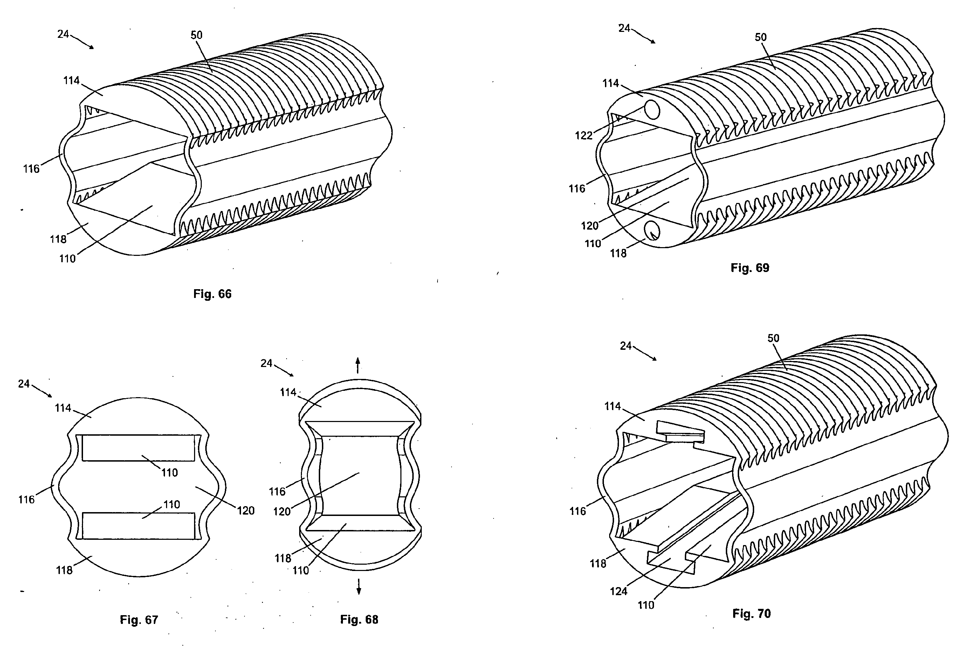

Figures 66 and 67 illustrates that the expandable section 24 can have a top wall 114 and a bottom wall 118 connected by two side walls 116. The top wall 114 and bottom wall 118 can have expandable thread 66. The side wall 116 can have expandable thread 66. The top wall 114 and/or bottom wall 118 can have one or more ramps 110 extending inwardly into the longitudinal channel 120 of the expandable section 24.

Figure 68 illustrates that in a radially expanded configuration, the top wall 114 and bottom wall 118 can translate radially outward, as shown by arrows. The side walls 116 can deform and/or translate radially inward.

Figure 69 illustrates that the top wall 114 and/or bottom wall 118 can have a manipulation channel 122 passing completely or partially therethrough in a substantially longitudinal direction. The manipulation channels 122 can be, for example, cylindrical.

Figure 70 illustrates that the top wall 114 and/or the bottom wall 118 can have longitudinal guide slots 124. The guide slots 124 can be in fluid communication with the longitudinal channel 122. The guide slots 124 can be parallel with the ramps 110.

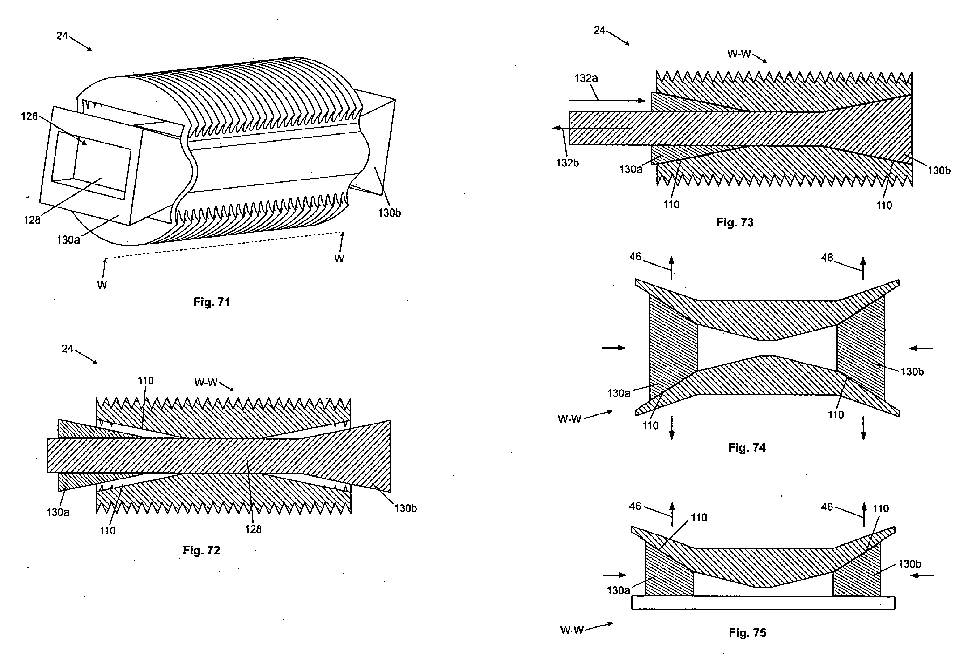

Figures 71 and 72 illustrate that a first wedge 130a and a second wedge 130b can be inserted into the longitudinal channel 122 of the expandable section 24. The second wedge 130b and/or first wedge 130a can be integral with the deployment rod 128. The first wedge 130a can have a longitudinal wedge channel 126. The deployment rod 128 can slidably attach to the first wedge 130a through the wedge channel 126. The first wedge 130a and second wedge 130b can have configurations that substantially match the respective ramps 110.

Figure 73 illustrates that the opposing compressive first and second translational forces 132 can be applied to the first wedge 130a and the deployment rod 128, respectively. The first and second wedges 130a, 130b can be deformably translated into the expandable section 24.

Figure 74 illustrates that the expandable section 24 can radially expand, for example near the ends of the expandable section 24 and/or to the length the wedges 130 are inserted.

Figure 75 illustrates that the expandable section 24 and wedges 130 can be configured to radially expand on only one side. For example, the wedges 130 can have angled slopes on one side of the wedge 130 and flat sides on the opposing side of the angled slopes. The expandable section 24 can have a wall 184 with tapered thickness on the side to be radially expanded, and a constant thickness wall 184, and/or a thicker wall 184 than the tapered wall 184, on the side opposite the tapered wall 184.

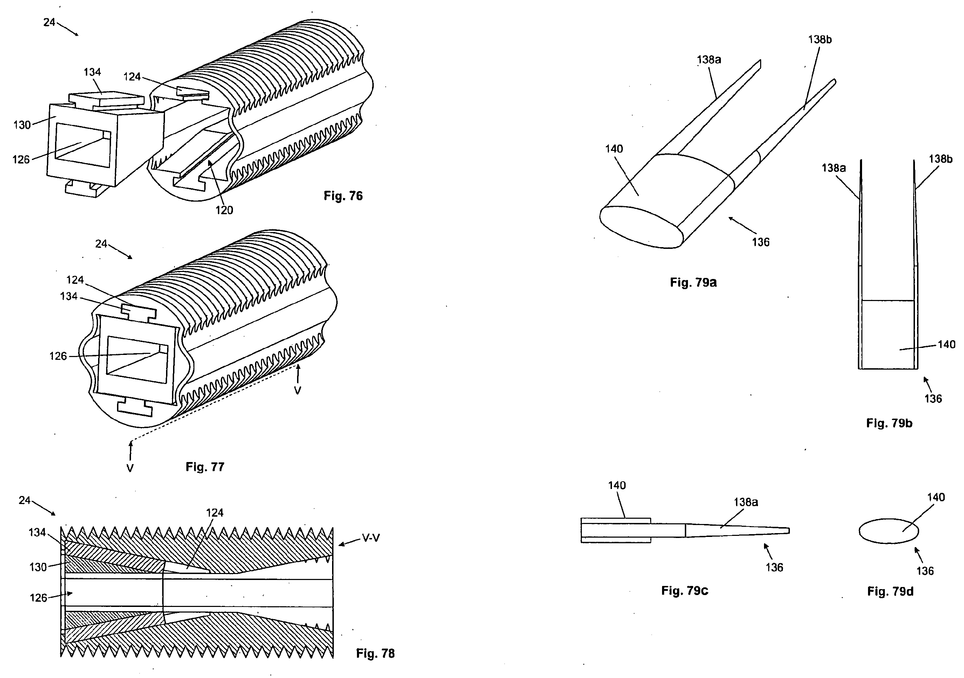

Figure 76 illustrates that the wedge 130 can have a wedge rail 134. The wedge rail 134 can align with and insert into the guide slot 124. Figures 77 and 78 illustrate that the wedge rail 134 can slidably attach to the guide slot 124.

Figures 79a through 79d illustrate that a manipulation tool 136 can have a base 140, a first leg 138a extending from the base 140, and a second leg 138b extending from the base 140. The legs 138 can be configured to fit into the manipulation channels 122 of the expandable section 24. The legs 138 can be used to insert into the manipulation channels 122 and manipulate (e.g., translation, rotation, deformation) the expandable section 24. Legs 138 can articulate with respect to the base 140. The leg 138 articulation can be controlled by controls (not shown) on the base 140, such as a handle 224 or trigger.

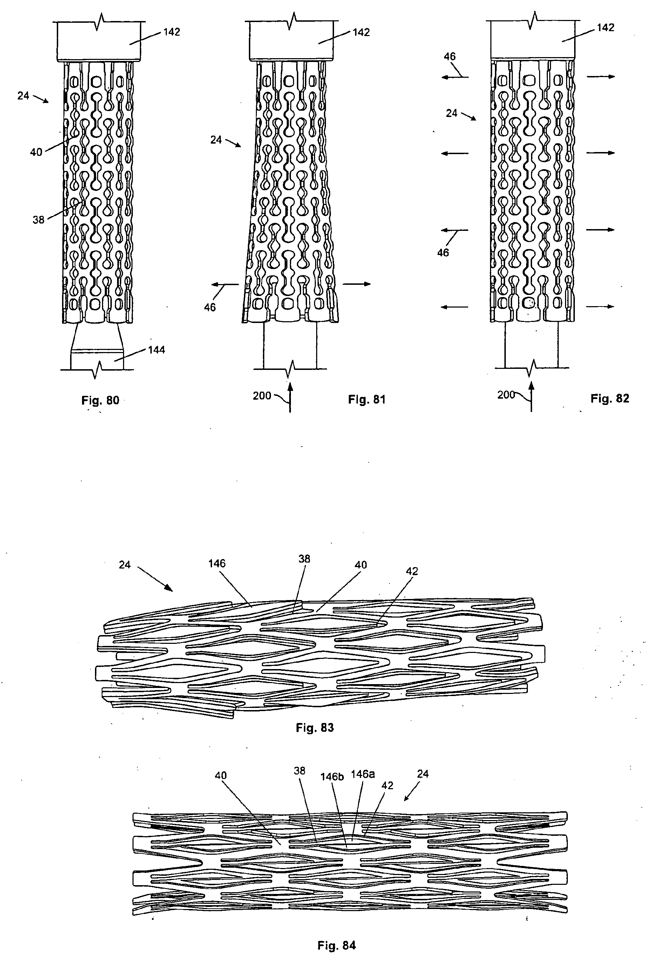

Figure 80 illustrates that a cone 144 or mandrel 188 can be translated into the longitudinal channel 120 of an expandable section 24 having struts 38 and joints 40. The expandable section 24 can have no hoops 36. The expandable section 24 can have an anvil 142 at the opposite end of the cone 144.

Figure 81 illustrates that the cone 144 can be forced 132 toward the anvil 142, and/or the anvil 142 can be forced toward the cone 144, resulting in longitudinal translation, as shown by arrow 200, of the cone 144 towards the anvil 142, through the longitudinal channel 120. The expandable section 24 over the cone 144, for example at the proximal end 34, can radially expand, as shown by arrows.

Figure 82 illustrates that the cone 144 can be longitudinally translated along the entire length of the expandable section 24. The cone 144 can be received in the anvil 142. The entire length of the expandable section 24 can radially expand, as shown by arrows. The expansion can be resilient and/or deformable. The cone 144 can be removed or left in place.

Figure 83 illustrates that the expandable section 24 can have plates 146 that can be integral with or attached to the joints 40 and/or struts 38. The plates 146 can be configured to be flexibly attached to or integral with the remainder of the expandable section 24. Each plate 146 can be configured to substantially cover each port 42.

Figure 84 illustrates that a first plate 146a and a second plate 146b can cover a port 42. The first plate 146a can extend from a first joint 40a adjacent to the port 42. The second plate 146b can extend from a joint 40 opposite to the first plate 146a.

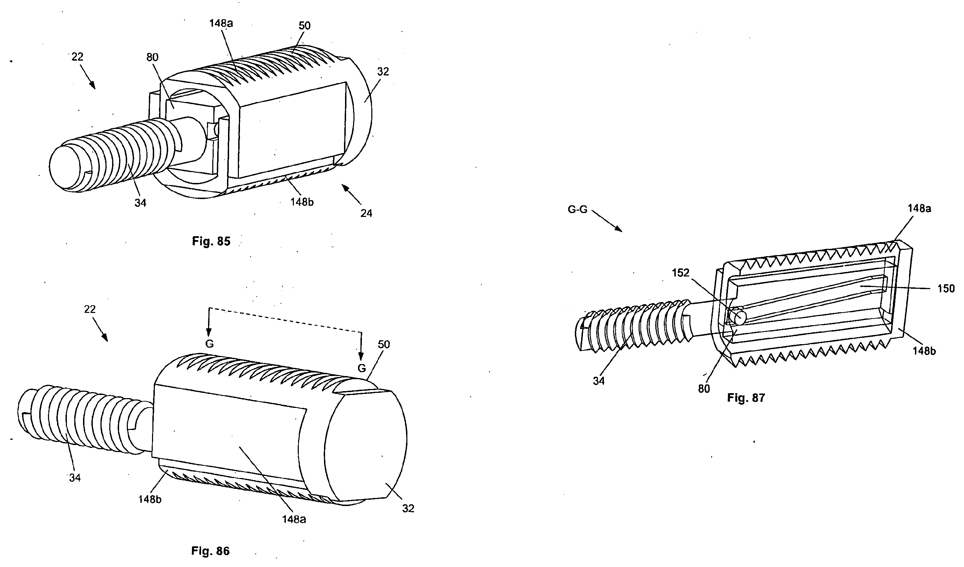

Figures 85 through 87 illustrate an expandable attachment device 22 that can have an expandable section 24 that can have a first expandable element 148a directly or indirectly slidably attached to a second expandable element 148b. For example, the first expandable element 148a can be slidably attached to the center shaft 80 to translate up when the center shaft 80 is translated distally, and the second expandable element 148b can be slidably attached to the center shaft 80 to translate down when the center shaft 80 is translated distally. When the expandable attachment device 22 is in a radially contracted configuration, the center shaft 80 can be substantially inside the expandable element 148. When the expandable attachment device 22 is in a radially expanded configuration, the center shaft 80 can be substantially outside the expandable element 148.

The first expandable element 148a can have the tip 32. The tip 32 can be pointed and/or flat. The first expandable element 148a can have thread 50 on a top side. The first expandable element 148a can have a peg 152(shown in Fig. 87) that can extend radially inward. The peg 152 can be configured to slide in a first track 150a on the side of the central shaft 80. The first track 150a can extend from being low distally to high proximally.

The second expandable element 148b can have thread 50 on a bottom side. The first expandable element 148a can have a peg 152 that can extend radially inward similar to that of the first expandable element 148a. The peg 152 can be configured to slide in a second track 150b on the side of the central shaft 80 opposite the side of the first track 150a. The first track 150a can extend from being high distally to low proximally.

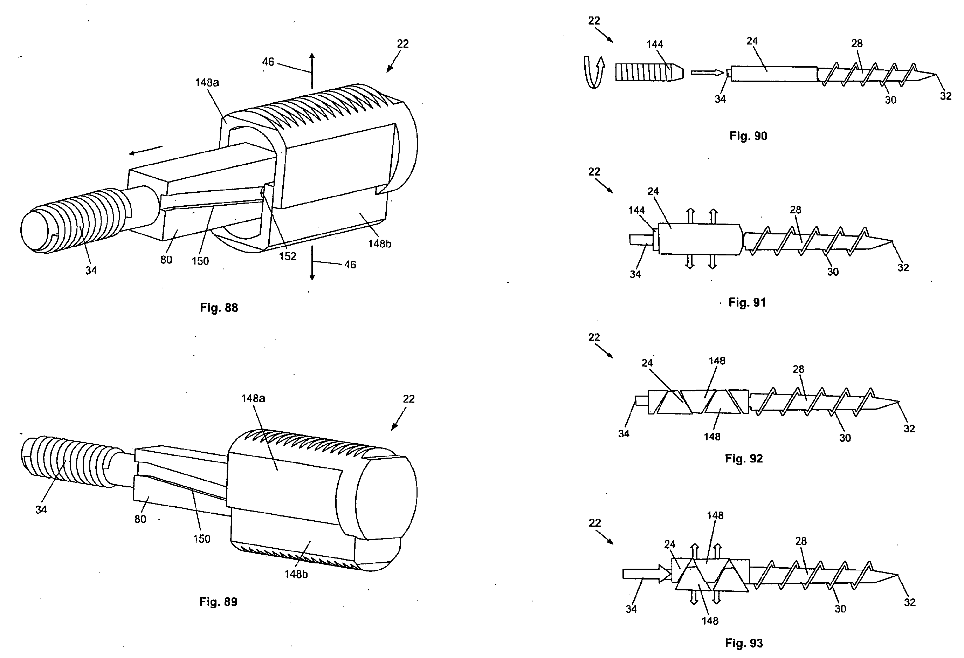

Figure 88 illustrates that when the expandable attachment device 22 is in a radially expanded configuration, the first expandable element 148a can be separated from the second element.

As the central shaft 80 is withdrawn from the expandable section 24, the peg 152 of the first expandable element 148a can be forced upward, forcing the first expandable element 148a upward. As the central shaft 80 is withdrawn from the expandable section 24, the peg 152 of the first expandable element 148a can be forced 132 upward, as shown by arrow in Figure 88, forcing the second expandable element 148b downward.

As the central shaft 80 is withdrawn from the expandable section 24, the peg 152 of the second expandable element 148b can be forced downward, forcing the second expandable element 148b upward, as shown by arrow in Figure 88.

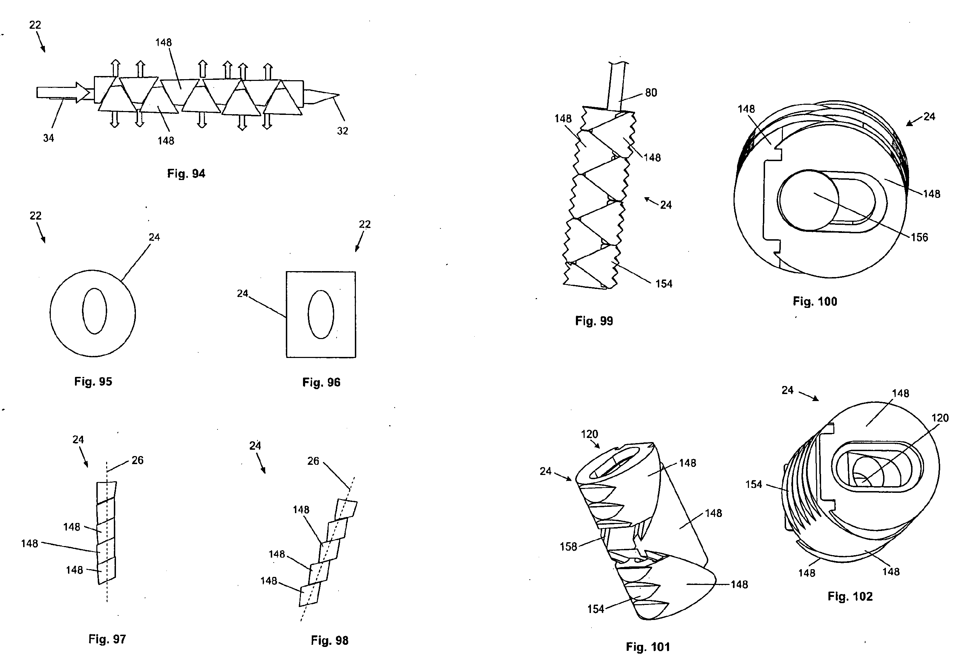

Figure 94 illustrates that the expandable element 142 devices can be substantially triangular from a lateral perspective. The expandable elements 142 can be slidably attached to each other. The expandable attachment device 22 can have multiple expandable elements 148. A compressive force 132, for example including a proximally directed force 132 applied to the proximal end 34 (as shown by arrow) and/or the distal expandable element 148, can force the expandable elements 148 to radially expand, as shown by arrows.

Figure 95 illustrates that the proximal end 34 of the expandable attachment device 22, for example the tip 32, can have a transverse cross-section that can be round, circular, oval, square, rectangular, triangular, or combinations thereof. The expandable section 24 can have a transverse cross-section that can be round, circular, oval, square, rectangular, triangular, or combinations thereof. Figure 96 illustrates a variation of the expandable section 24.

Figure 97 illustrates that the expandable section 24 in the radially contracted configuration can have a straight expandable section axes 26. Figure 98 illustrates that the expandable section 24 in a radially expanded configuration can have a straight or curved expandable section axis 26, and/or that the expandable section axis 26 can be at an angle with respect to the expandable section axis 26 in the radially contracted configuration.

Figures 99 and 100 illustrates that the expandable section 24 can have a series of expandable elements 148 having a slidably attached center shaft 80 therethrough. The center shaft 80 can have a center shaft anchor 156. The center shaft anchor 156 can have a larger diameter than the diameter of the longitudinal channel 120. Teeth 154 can radially extend from the expandable elements 148, for example from at least opposite sides of alternating expandable elements 148, as shown.

Figures 101 and 102 illustrate that the expandable elements 148 can have guide rails 158. The guide rails 158 can slidably attach to receiving elements on adjacent expandable elements 148. The longitudinal channel 120 in at least every other expandable element 148 can be elongated in the transverse direction.

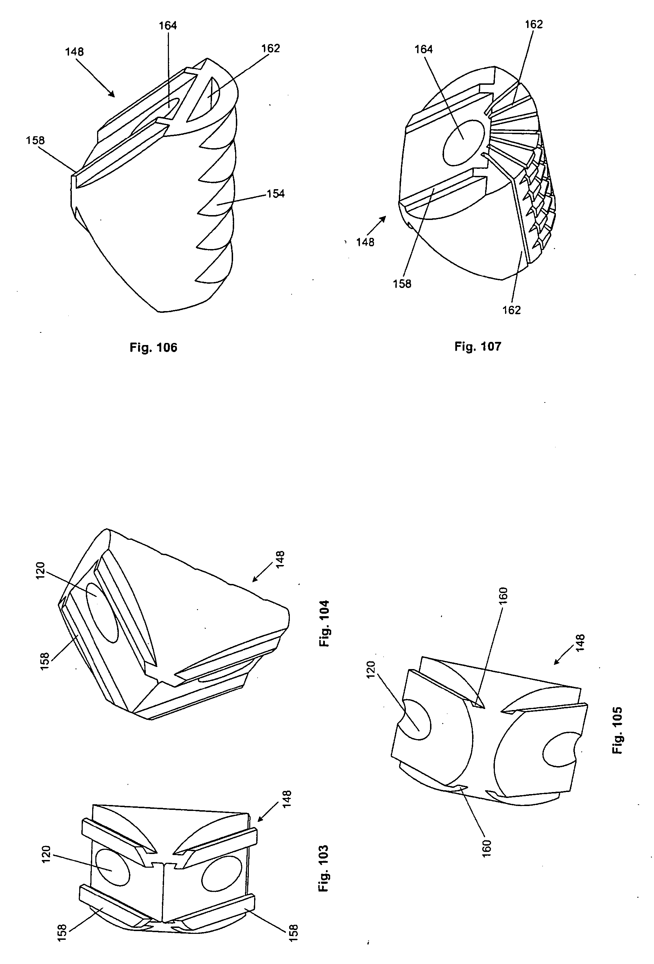

Figures 103 and 104 illustrate that the expandable element can have one or two guide rails 158 on each surface adjacent to another expandable element when assembles. The cross-section of the longitudinal channel 120 in an individual expandable element can be, for example, circular, oval, square, rectangular, or combinations thereof.

Figure 105 illustrates that the expandable element 148 can have one, two or more guide grooves 160 on each surface adjacent to another expandable element when assembled. The guide grooves 160 can be configured to slidably attach to the guide rails 158.

Figure 106 illustrates that the expandable element 148 can have one or more contouring channels 162. The contouring channels 162 can be a defined, substantially closed volume within the expandable element 148. The contouring channels 162 can deform, for example, due to force 132 applied against the teeth 154 during use. When deformed, the contouring channel 162 can, for example, reduce the stress applied on the neighboring tissue when implanted compared to the expandable element 148 in a non-deformed configuration.

Figure 107 illustrates an expandable element 148 having a number of contouring channel 162 extending radially away from the expandable element channel 164. The contouring channels 162 can be configured as slots open to the outside of the expandable element 148.

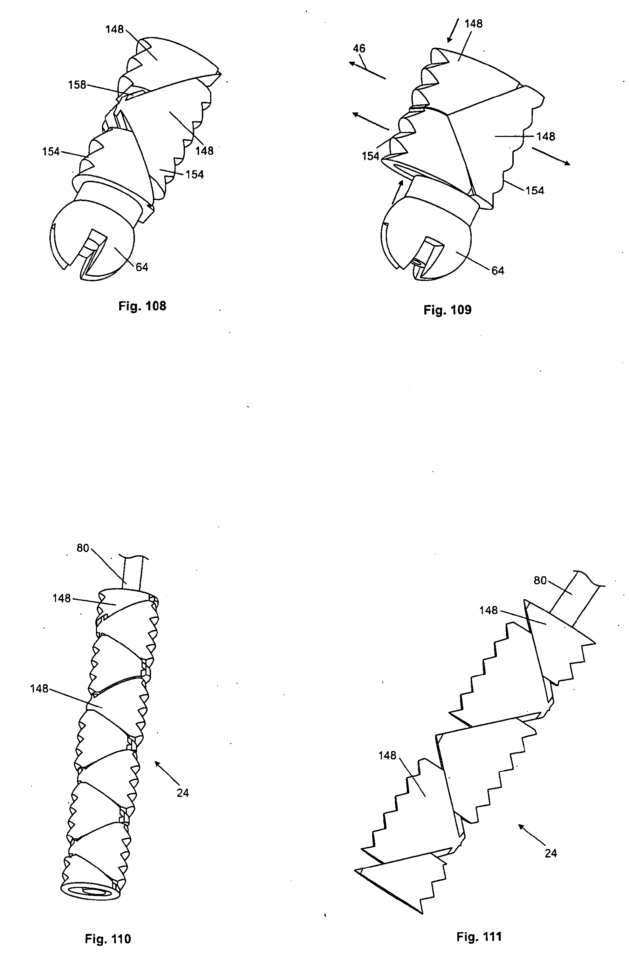

Figure 108 illustrates that the proximal end cap 64 can be distal to the most distal expandable element 148. For example, the proximal end cap 64 can be, or be attached to, the center shaft anchor 156.

Figure 109 illustrates that a longitudinally compressive force, as shown by arrow, can be delivered through the proximal end cap 64. The expandable elements 148 can then radially expand, as shown by arrows.

Figures 110 and 111 illustrate the expandable section 24 having nine and five expandable elements 148, respectively.

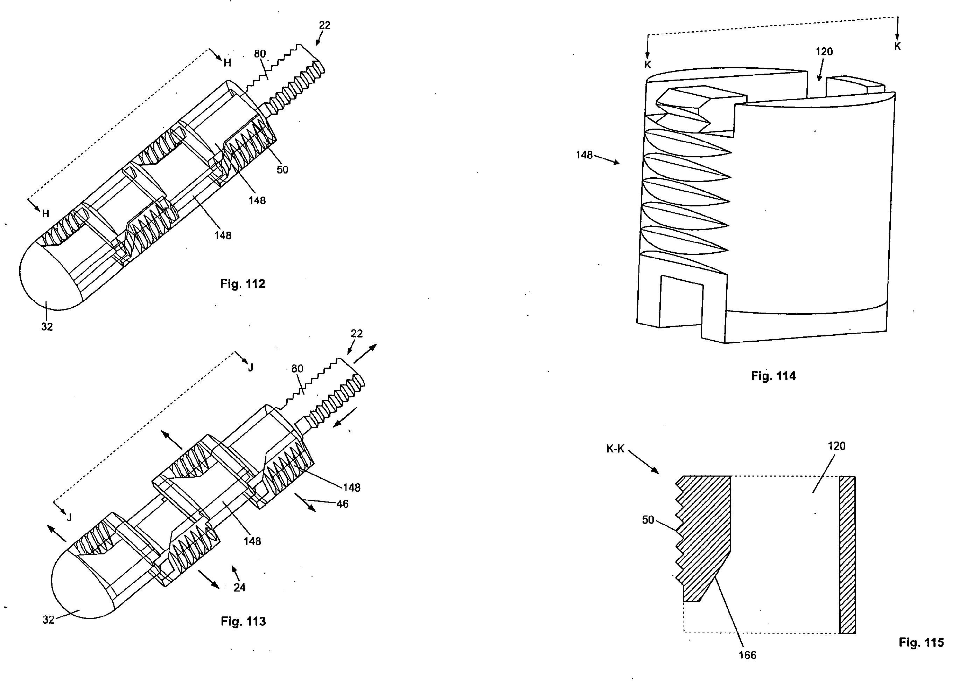

Figures 112 and 113 illustrates that the center shaft 80 can be configured to have one or more alternately oppositely facing integral wedges 112. The expandable section 24 can have one or more expandable elements 148. The expandable elements 148 can have guide rails 158 on the proximal ends and guide grooves 160 on the distal ends 34. The guide grooves 160 and guide rails 158 can constrain relative motion between the expandable elements 148 to a single degree of freedom (e.g., lateral motion). The internal surfaces of the expandable elements 148 can have alternately oppositely facing internal ramps 166 that can be configured to abut the integral wedges 112.

Figure 113 illustrates that the center shaft 80 can be translated relative to the expandable section 24, for example with the center shaft 80 being translated out of the expandable section 24. The expandable elements 148 can then radial expand in opposite directions as the adjacent expandable elements 148, as shown by arrows.

Figure 114 illustrates that the expandable element 148 can have one or two guide grooves 160 in the proximal end 34 of the expandable element 148. The guide grooves 160 can be notches in the wall around the longitudinal channel 120. The expandable element 148 can have one or two guide rails 158 at the proximal end of the expandable element 148. The guide rails 158 can be configured to slidably attach to the guide grooves 160 when one expandable element 148 in stacked on another expandable element 148.

Figure 115 illustrates that the internal ramp 166 can be a slope on the internal surface of the longitudinal channel 120. The thread 50 can be on a single side of the expandable element 148.

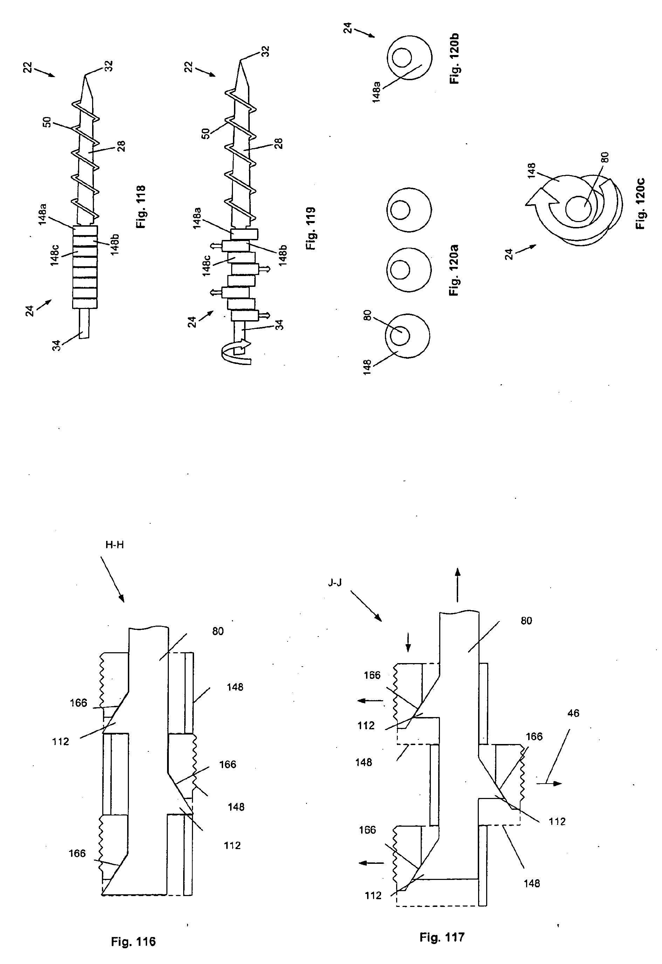

Figures 116 and 117 illustrate that when the integral wedges 112 of the center shaft 80 press into the internal ramps 166 of the expandable elements 148, as shown by arrows in Figure 117, the expandable elements 148 can be pushed radially outward by the integral wedges 112, as shown by arrows.

Figure 118 illustrates that the expandable section 24 can have first, second, third and more expandable elements 148a, 148b, 148c that can be cams or other offset-rotation elements. Figure 119 illustrates that the proximal end 34 can be rotated, as shown by arrow. The expandable elements 148 can then radially translate or expand, as shown by arrows. The expandable elements 148 can translate at different timings.

Figure 120a illustrates that the expandable elements 148 can have a center shaft 80 extending through the expandable elements 148. The center shaft 80 can be offset from the center of area of the expandable element 148 in the plane transverse to the expandable attachment device 22 axis.

Figure 120b illustrates that the expandable section 24 in a radially contracted configuration can have all of the expandable elements 148 substantially aligned along the expandable attachment device 22 axis.

Figure 120c illustrates that the expandable section 24 can be radially expanded by rotating the center shaft 80 and/or rotating the expandable elements 148 around the center shaft 80. The cam expandable elements 148 can splay and radially expand.

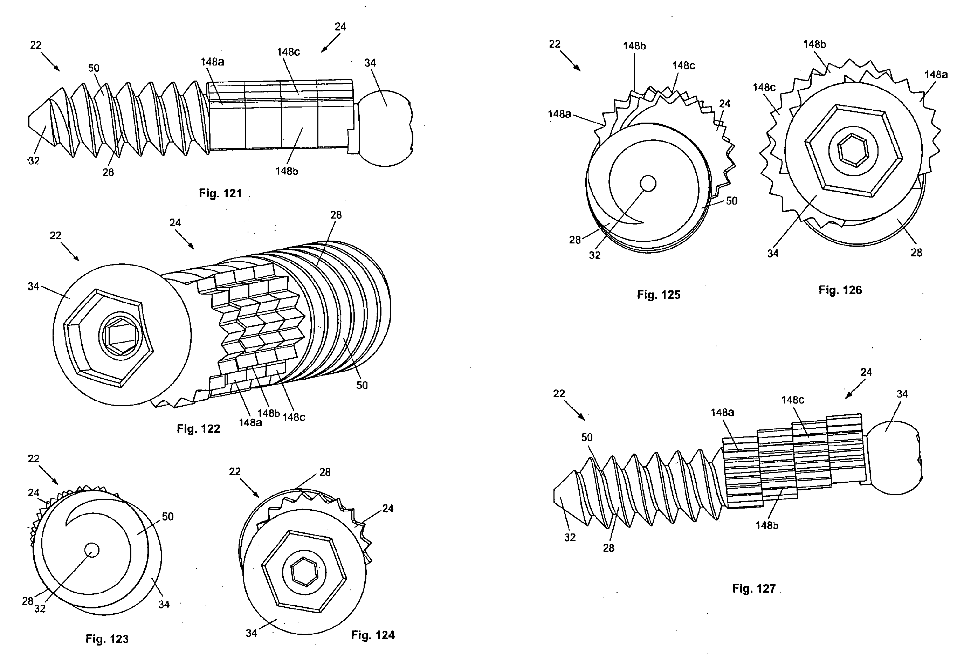

Figures 121 illustrates that the expandable attachment device 22 can have multiple expandable elements 148 eccentrically attached to a center shaft 80, and/or with lobed configurations.

Figures 122 through 124 illustrate that the expandable attachment device 22 can have one through four expandable elements 148 eccentrically attached to a center shaft 80 (not shown). The expandable elements 148 can have teeth 154 radially extending from the expandable elements 148.

Figures 125 through 127 illustrate the expandable attachment device 22 with eccentrically attached expandable elements 148 in a radially expanded configuration.

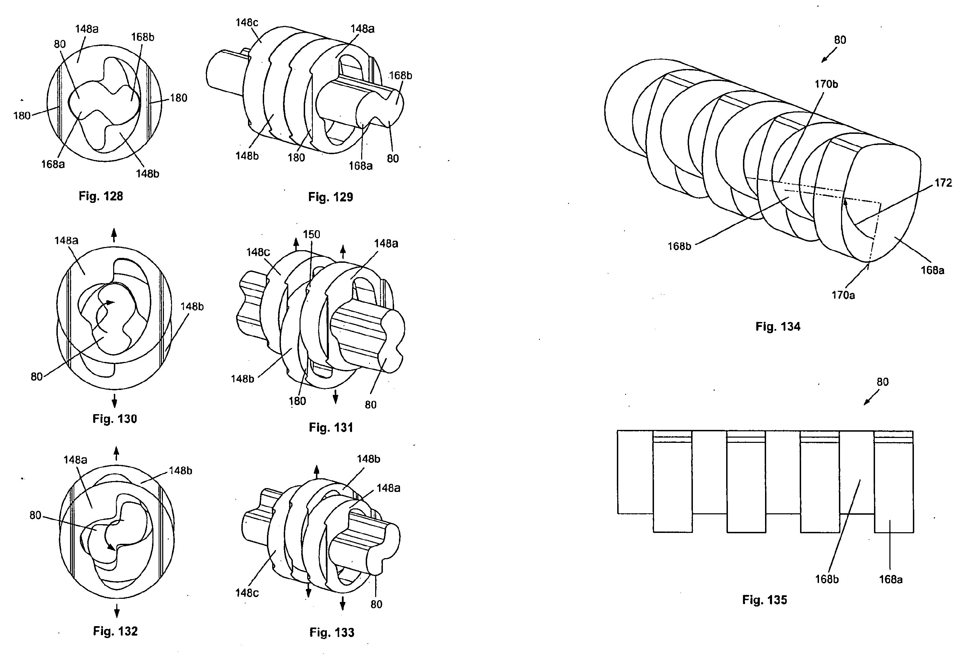

Figures 128 and 129 illustrate that the expandable section 24 can have a first, second and third expandable element 148a, 148b, 148c. The expandable elements 148 can be slidably attached by interlocking rails 110 and tracks 150. The rails 110 and tracks 150 can constrain relative motion between adjacent expandable elements 148 to one degree of freedom (e.g., vertical relative motion).

The expandable elements 148 can have longitudinal channels 120 configured, for example as shown, to receive a multi-lobed center shaft 80 and be controllable as shown in Figures 128 through 133. The configuration of the longitudinal channel 120 in each expandable element 148 can be the same or different as the other expandable elements 148. For example, the first expandable element 148a and the third expandable element 148c can have substantially identically configured longitudinal channels 120. The second expandable element 148b can have a longitudinal channel 120 configured to be a horizontally reversed configuration of the longitudinal channel 120 of the first expandable element 148a. The second expandable element 148b can have a longitudinal channel 120 configured to be-about a 180° rotation of the longitudinal channel 120 of the first expandable element 148a. The center shaft 80 can have a first lobe 168a and a second lobe 168b.

Figures 130 and 131 illustrate that the center shaft 80 can be rotated, as shown by arrow. When the center shaft 80 is rotated, the lobes 168 can exert forces 132 against the expandable elements 148. The expandable elements 148 can be translated in a direction substantially perpendicular to the longitudinal axis of the center shaft 80. For example, the first and third expandable elements 148a, 148b, 148c can translate toward the up, as shown by arrows. The second expandable element 148b can translate down, as shown by arrows.

Figures 132 and 133 illustrates that the center shaft 80 can be rotated in the opposite direction as shown in Figures 130 and 131. The expandable elements 148 can translate in the opposite direction as shown from Figure 131.

Figures 134 and 135 illustrate a center shaft 80 that can have alternating first lobes 168a and second lobes 168b along the length of the center shaft 80. The first lobes 168a can have a first lobe axis 170a. The second lobes 168b can have a second lobe axis 170b. When viewed in the same plane, the angle between the first lobe axis 170a and the second lobe axis 170b can be a lobe angle 172. The lobe angle 172 can be from about 90° to about 180°. The first lobes 168a can be actuated in an opposite rotational direction than the second lobes 168b.

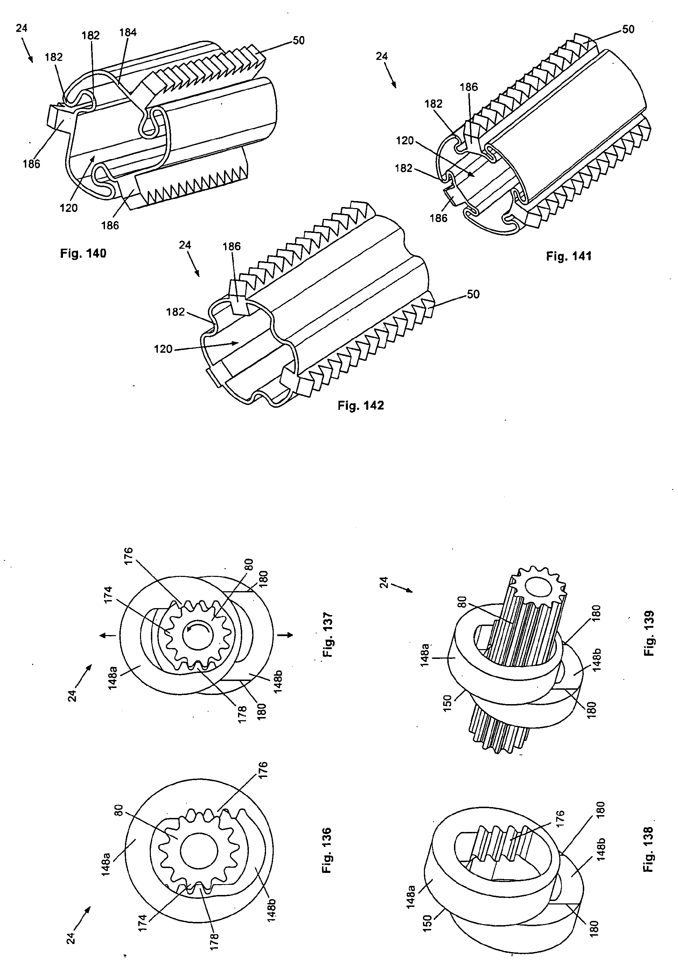

Figure 136 illustrates an expandable section 24 that can have a first expandable element 148a that can translate in the opposite direction of the second expandable element 148b when the center shaft 80 is rotated. The first expandable element 148a can have first element teeth 176a. The second expandable element 148b can have second element teeth 176b. The element teeth 176 can extend radially inward in the longitudinal channel 120. The first element teeth 176a can be on the opposite side of the longitudinal channel 120 as the second element teeth 176b. The center shaft 80 can have gear teeth 174 extending radially outward. The gear teeth 174 can engage the first element teeth 176a can the second element teeth 176b.

Figures 137 through 139 illustrate that when the center shaft 80 is rotated, the first expandable element 148a can translate up at the same rate that the second expandable element 148b can translate down.

Figure 140 illustrates an expandable section 24 that can have thread 50 or teeth 154 on one, two, three or more spines 186 extending radially from the wall 184 of the expandable section 24. In a radially contracted configuration, the wall 184 can have multiple folds 182, for example two folds 182 between each two adjacent spines 186. The folds 182 can be unevenly spaced between the adjacent spines 186.

Figure 141 illustrates that the wall 184 can have two folds 182 between each two adjacent spines 186. The folds 182 can be evenly spaced between the adjacent spines 186.

Figure 142 illustrates that the walls 184 can have one fold 182 between adjacent spines 186. The spines 186 can extend radially inward and/or outward from the wall 184.

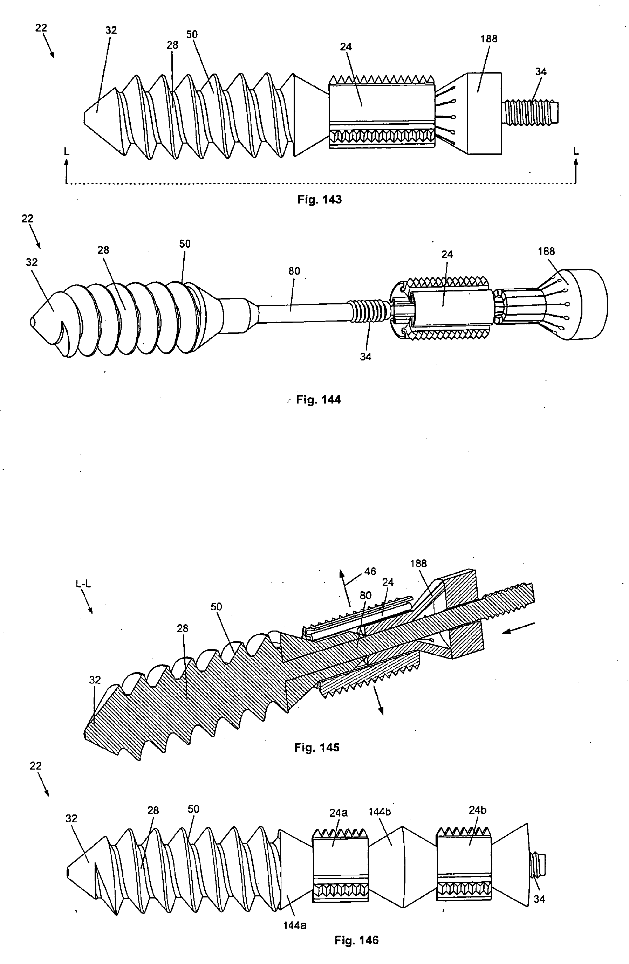

Figures 143 and 144 illustrate that the expandable section 24(shown for exemplary purposes as the expandable section 24 of Figure 141) can be loaded on the center shaft 80 of an expandable attachment device 22. The expandable section 24 can be placed between a first cone 144a and a second cone 144b on the expandable attachment device 22. The expandable attachment device 22 can have a mandrel 188. The second cone 144b can be part of the mandrel 188.

Figure 145 illustrates that the mandrel 188 can be pushed, as shown by arrow, toward the expandable section 24. The expandable section 24 can radially expand as shown by arrow.

The proximal end 34 can be configured to attach to a separate device, such as a fixation rod 14 or plate 220. The proximal end 34 can be threaded 50.

Figure 146 illustrates that the expandable attachment device 22 can have a first expandable section 24a and a second expandable section 24b. Each expandable section 24 can be between a first cone 144a and a second cone 144b, and can be radially expanded as described herein, including as shown in Figure 145.

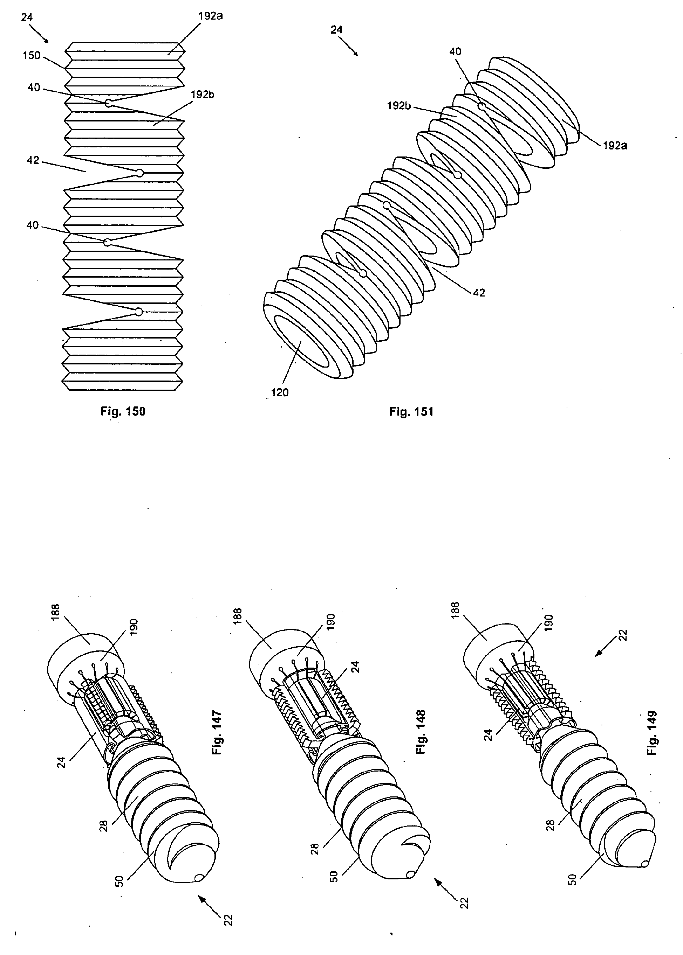

Figures 147 through 149 illustrate the expandable sections 24 of Figures 140 through 142, respectively, loaded on the center shaft 80 of the expandable attachment device 22.

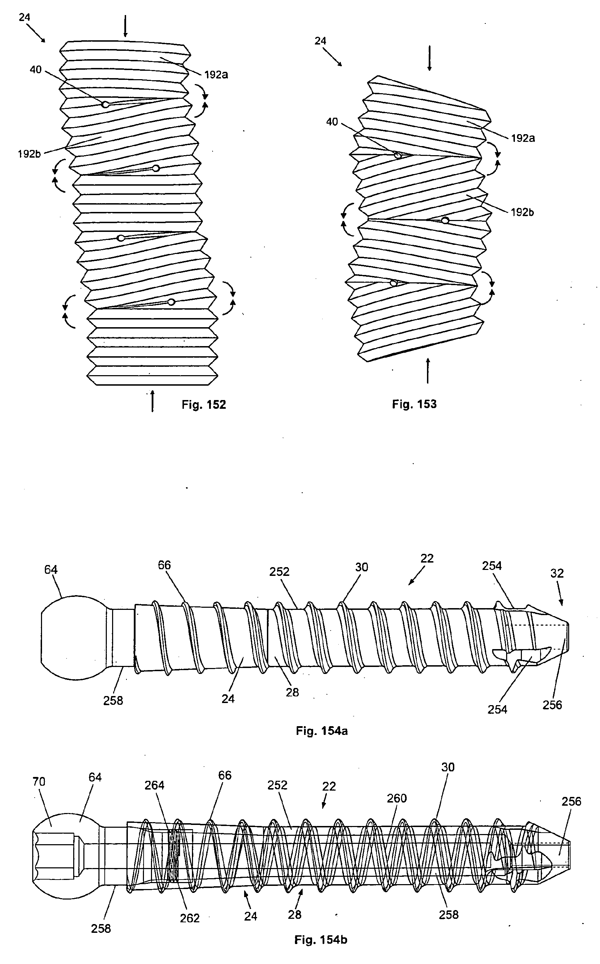

Figures 150 and 153 illustrate that the expandable section 24 can have about four angled ports 42. Each port 42 can have a joint 40. Between two adjacent ports 42 can be an individual expandable segment 192, for example the first expandable segment 192a and the second expandable segment 192b, as shown.

Figure 152 illustrates that a longitudinally compressive force, as shown by arrows, can be applied to the expandable section 24. The expandable segments 192 can rotate, as shown by arrows, around the adjacent joints 40. The ports 42 can close. In the radially expanded configuration, the expandable section 24 can have a proximal end 34 shifted laterally from the proximal end.

Figure 153 illustrates that the expandable section 24 can have larger ports 42 and/or the expandable section 24 can be over compressed, causing deformation after the ports 42 have closed. The proximal end 34 and the proximal end of the expandable section 24 can be laterally aligned.

Figures 154a and 154b illustrate that the attachment device 22 can have an outer shell 252 and an inner structure 258. The inner structure 258 can have an end cap 64 and a shaft extending from the end cap 64. When the device 22 is in an assembled configuration, the shaft can be in the outer shell 252. The end cap 64 can have the shaft deployment tool attachment 70.

A hollow channel 260 can extend partially or completely along the longitudinal axis of the device 22. The hollow channel 260 can be in fluid communication with the end cap 64, for example with the shaft deployment tool attachment 70.

The outer shell 252 can have an expandable section 24 and an unexpandable section 28. The expandable section 24 can be expanded or unexpanded during normal use (i.e., depending on the variation, the expandable section 24 may or may not expand).

The expandable section 24 can have expandable thread 30. The expandable thread 30 can be expanded or unexpanded during normal use.

The tip 32 can be sharpened (e.g., traumatic), blunted (e.g., atraumatic) a combination of sharpening with a flat terminal end (as shown), or other combinations thereof. The tip 32 can be configured to fixedly attach or seat the expandable attachment device 22 in bone 228.

The device 22 cam have one of more cutting teeth 254. For example, the device can have cutting teeth 254 near the terminal end of the device. The cutting teeth 254 can extend from the outer shell 252 surface radially away from the longitudinal axis and/or the cutting teeth 254 can be formed by the removal of a portion of the radially exterior external wall near the tip of the outer shell 252 (e.g., without increasing the radius of the cutting with respect to the adjacent wall of the outer shell 252). The cutting teeth 254 can extend longitudinally along a length of the outer shell 252. The cutting teeth 254 can be configured to cut through bone.

The device 22 can have one or more distal port 256s. The distal port 256 can in fluid communication with the hollow length. The hollow channel 260 and distal port 256 can be configured to allow the flow of filler therethrough. A source of filler, such as any material described herein in a flowable, morselized, or otherwise small enough particle size, or combinations thereof, can be in fluid communication with the proximal end of the hollow length. The filler can be delivered under pressure to the hollow length.

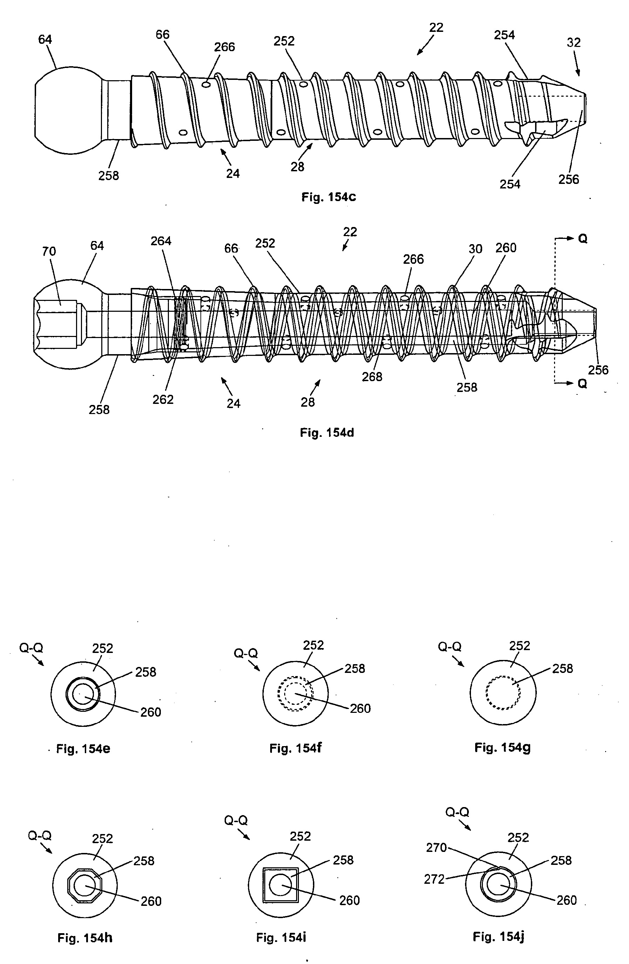

Figures 154c and 154d illustrate that the attachment device 22 can have one or more flow ports 266. The flow ports 266 can be in fluid communication with the cannulated hollow length of the inner structure 258. The flow ports 266 can pass through the wall of the outer shell 252 and/or the inner structure 258. The flow ports 266 through the wall of the inner structure 258 can align with the flow ports 266 through the wall of the outer shell 252 when the device 22 is in a filler delivery configuration. The flow ports 266 can be randomly or uniformly distributed along and along the device 22. For example, the flow ports 266 can be angularly positioned with respect to the longitudinal axis at 0°, about 120°, and about 240° relative angles when a flow port 266 is set as 0°.

A filler, locking or setting fluid, epoxy, any material disclosed herein, or combinations thereof (referred to collectively herein as "filler"), can be delivered through the shaft deployment tool attachment 70 and/or a separate filler intake port in fluid communication with the hollow length. The filler can flow through the hollow length The filler can exit the hollow length through the flow ports 266 and/or the distal port 256.

Figure 154e illustrates that the outer shell 252 and inner structure 258 can be co-axial cylinders. The gap between the outer shell 252 and the inner structure 258 can act a substantially fluid tight interface. The inner structure 258 can be axially and angularly (i.e., rotationally) slidable with respect to the outer shell 252. The hollow length can extend to the distal terminal end of the outer shell 252. The hollow length can be in fluid communication with the environment distal to the outer shell 252 at the distal terminal end of the outer shell 252.

Figure 154f illustrates that the inner structure 258 and hollow length can terminate before reaching the distal terminal end of the outer shell 252. The distal terminal end of the outer shell 252 can obstruct the hollow length from being in fluid communication with the environment distal to the outer shell 252 at the distal terminal end of the outer shell 252. A filler can be delivered through the hollow length that can be delivered to the treatment site through flow ports 266 in the lateral wall of the outer shell 252, but not through the distal end of the outer shell 252.

Figure 154g illustrates that the device can have no hollow length.

Figure 154h illustrates that the inner surface of the outer shell 252 can have a substantially hexagonal transverse cross-section. The outer surface of the inner structure 258 can have a substantially hexagonal transverse cross-section. The transverse cross-section of the outer surface of the outer shell 252 can be hexagonal or circular. The transverse cross-section of the inner surface of the inner structure 258 can be hexagonal or circular.

Figure 154i illustrates that the inner surface of the outer shell 252 can have a substantially square transverse cross-section. The outer surface of the inner structure 258 can have a substantially square transverse cross-section. The inner surface of the outer shell 252 and the outer surface of the inner structure 258 can have triangular, pentagonal or other polygonal transverse cross-sections.

Figure 154j illustrates that the inner structure 258 can have a longitudinal inner slot 272. The outer shell 252 can have a longitudinal outer rail 270. The inner slot 272 can be angularly aligned with the outer rail 270. The outer rail 270 can be longitudinally slidably received by the inner slot 272. The inner slot 272 and outer rail 270 can interface such that the inner structure 258 can be inserted into the outer structure at one (or more, for example if there are substantially equal-size and equal-shape outer rail 270s and inner slot 272s on opposite sides of the inner structure 258 and outer shell 252) angles with respect to the outer structure about the longitudinal axis.

Figures 154h through 154j illustrate that the inner structure 258 can be axially slidable with respect to the outer shell 252. The inner structure 258 can be substantially rotationally fixed with respect to the outer shell 252. The sides of the hexagonal or square (or triangular, pentagonal, or other polygonal) transverse cross-sections, and the rail and slot of Figure 154j can be detents to interference fit in the rotational degree of freedom about the longitudinal axis (i.e., perpendicular with the plane shown of Figures 154h through 154j).

A torque can be applied to the inner structure 258 that can then be transferred through the inner structure 258, through the detents or otherwise, to the outer shell 252. A torque can be applied to the outer shell 252 that can then be transferred through the outer shell 252, through the detents or otherwise, to the inner structure 258.

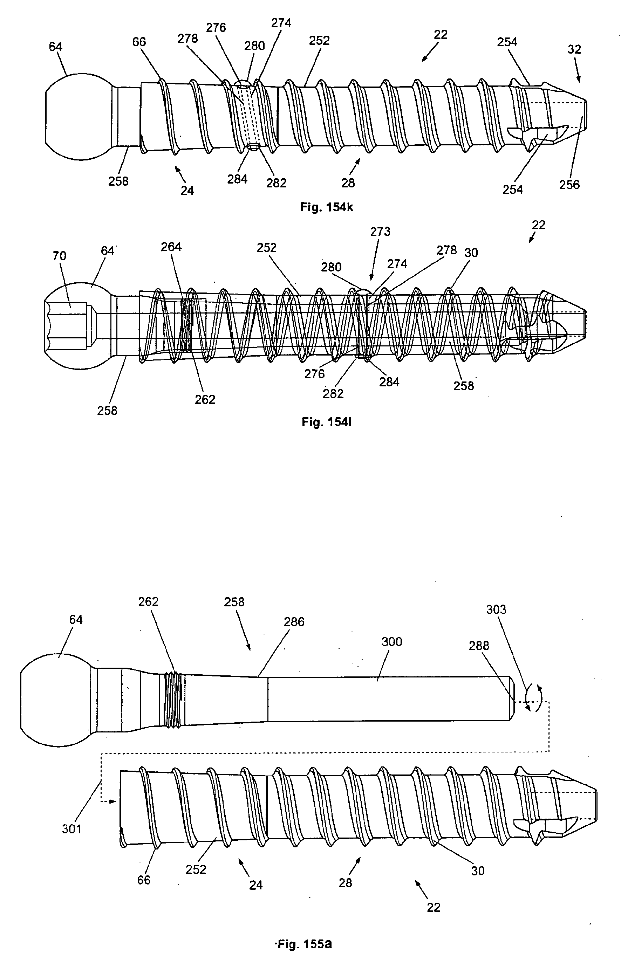

Figures 154k and 154l illustrate that the attachment device 22 can have a torque pin 273 inserted partially or completely through a torque pin port 276 in the outer shell 252 and the inner structure 258. The attachment device can have a torque pin channel 274. The torque pin 273 can be inserted through the torque pin channel 274.

The torque pin 273 can have a torque pin head 280 and a torque pin shaft 278. The torque pin head 280 can have a wider diameter than the torque pin channel 274. The torque pin shaft 278 can have a smooth, textured, threaded wall or combinations thereof. The torque pin 273 can be slid or screwed through the torque pin channel 274.

When the torque pin distal end 284 exits the torque pin channel 274, a torque pin clasp 282 can be attached or a collet or deformable radial expansion (e.g., by longitudinally crushing) of the torque distal end can be attached or formed to be wider than the diameter of the torque pin channel 274.

The torque pin channel 274 can be threaded or smooth. The torque pin channel 274 can pass through the walls of the outer shell 252 and the inner structure 258. The torque pin channel 274 can be open to the hollow length and/or the torque pin channel 274 can have a wall circumscribing the torque pin channel 274 within the hollow length. The torque pin 273 can be configured to transfer torque between the inner structure 258 and the outer shell 252.

Figures 155a illustrates that the inner structure 258 can have a shaft 300. The shaft 300 can have a shaft terminal end 288. The shaft can fixedly or releasably attach to the inside and/or outside of the outer shell 252. The inner structure 258 can be rotated, as shown by arrows 303, relative to the outer shell 252. The inner structure 258 can be translated, as shown by arrow 301, relative to the outer shell 252 into the outer shell 252. The structure outer thread 262 and/or shell inner thread 264 can engage and/or a single aforementioned thread can engage a smooth wall on the corresponding element. For example, the inner structure 258 can be screwed into the outer shell 252.

The inner structure 258 transverse cross-section and the outer shell 252 transverse cross-section can be substantially the same or different shapes. For example, the inner structure 258 transverse cross-section and the outer shell 252 transverse cross-section can be circular, oval, square, rectangular, triangular, other polygonal shapes, or combinations thereof. An inner structure 258 with a non-similarly shaped transverse cross-section relative to the transverse cross-section of the outer shell 252 can create pressure risers (e.g., locations of higher compressive pressure) where the non-similarly shaped cross-sections initially interface.

The attachment device can have two, three, or more separatable elements. For example, the attachment device can have an inner structure 258, an outer shell 252, a torque locking pin 273 (e.g., as shown in Figures 154k and 154l), or combinations thereof.

The inner structure 258 can have one or more inner structure shoulders 286. The inner structure shoulders 286 can be at the proximal end, distal end, central portion, or combinations thereof, of the shaft. The inner structure shoulder 286 can have a sloped wall. For example, the inner structure shoulder 286 can have, as the shoulder is more proximal, an increasing radius with respect to the longitudinal axis of the inner structure 258. The outer shell 252 can radially expand when the shoulder is forced into the hollow length of the outer shell 252.

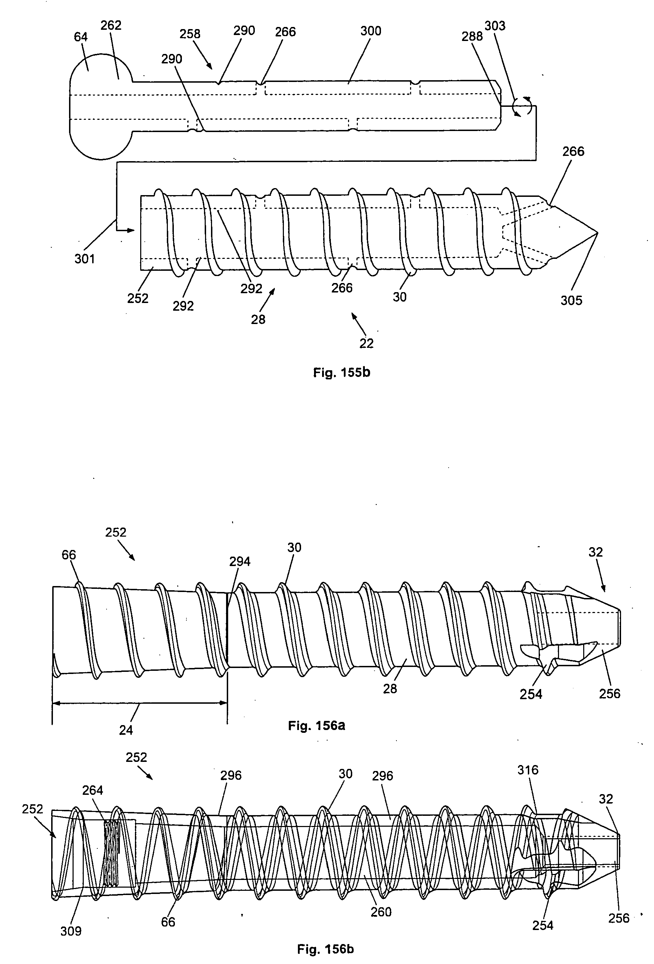

Figure 155b illustrates that the flow ports 266 in the outer structure can align with some or all of the flow ports 266 in the inner structure 258 when the device is in an assembled and deployed configuration. The outer shell 252 can have a distal tip 305 that can have a sharp, flat, or bullet tip. One or more flow ports 266 can emerge at or near the distal tip 305. The flow ports 266 can be in fluid communication with the hollow length.

The outer surface of the inner structure 258 can have one or more inner detent 290s. The inner detent 290s can be aligned with each other and/or staggered longitudinally and/or angularly about the inner structure 258. The inner detent 290s can be divets, slots, recepticles, indetations, or combinations thereof.

The inner surface of the outer structure can have one or more outer detent 292s. The outer detent 292s can be aligned with each other and/or staggered longitudinally and/or angularly about the outer shell 252. The outer detent 292s can be rails, nubs, bumps, lumps, protuberance, or combinations thereof.

The outer detent 292s can be substantially aligned with the inner detent 290s when the device is in an assembled and deployed configuration. The outer detent 292s can intereference fit with the inner detent 290s to substantially rotationally fix the inner structure 258 to the outer shell 252 with respect to the longitudinal axis.

The outer surface of the inner structure 258 can have a substantially hexagonal (or other polygonal) transverse cross-sectional configuration. The outer shell 252 can be crimped onto the inner structure 258. The outer detent 292s can be formed by the crimping. The outer detent 292s can interface with the sides of the hexagonal transverse cross-sectional configuration of the outer surface of the inner structure 258. For example, the outer detent 292s can intereference fit with the sides of the hexagonal transverse cross-sectional configuration to substantially rotationally fix the inner structure 258 to the outer shell 252 with respect to the longitudinal axis.

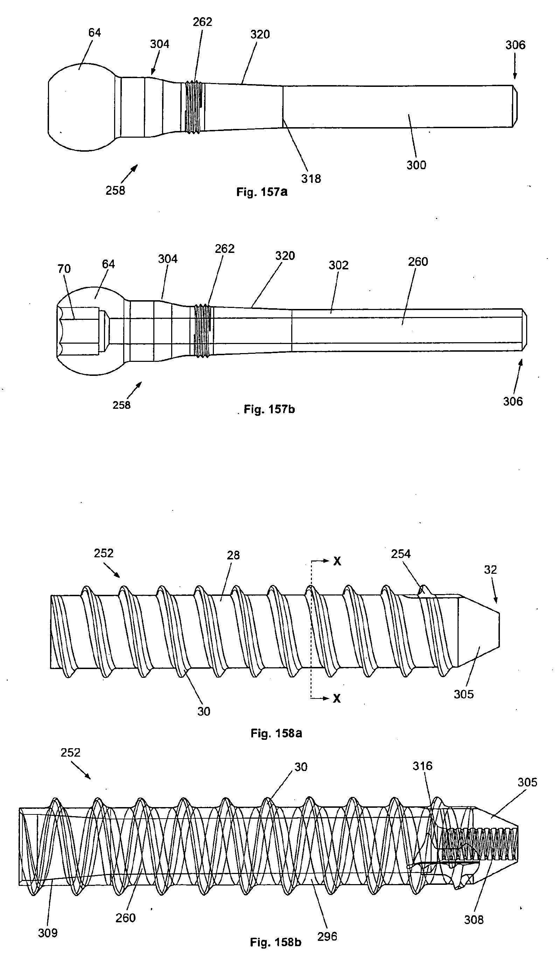

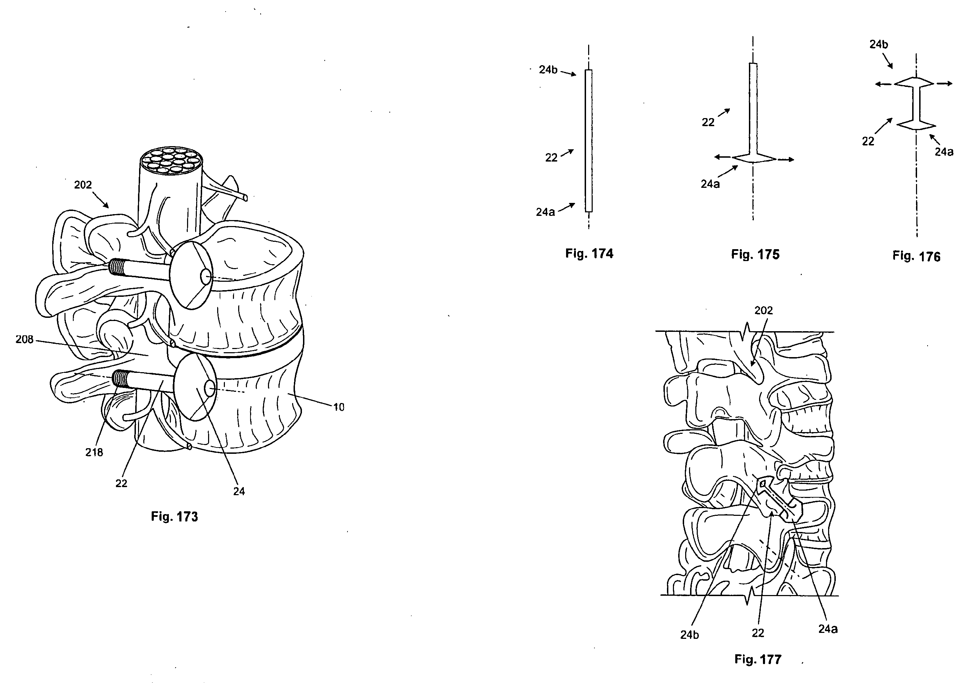

Figures 156a and 156b illustrate that the outer shell 252 (and/or inner shell, as shown in Figures 157a and 175b, inter alia) can be made of one, two or more attachable sections, for example the expandable section 24 and the unexpandable section 28. The expandable section 24 can be attached to the unexpandable section 28 at an attachment outer seam 294. The attachment outer seam 294 can have a welding (e.g., a laser welding), molding seam, heat seal, epoxy, or combinations thereof.

Figure 156b illustrates that the outer shell 252 can have hollow length. The hollow length can be configured to releasably or fixedly attach to the shaft and/or other elements of the inner structure 258.

The outer shell 252 can have a distal shell inner stop 316. The distal shell inner stop 316 can be a detent. The inner structure tip 306 can interference fit against the distal shell inner stop 316, for example to prevent the inner structure 258 from distally exiting the outer shell 252 during insertion. The distal shell inner stop 316 can have a similar shape to the inner structure tip 306. The inner structure tip 306 can seat in the distal shell inner stop 316.

The outer shell 252 can have shell inner threads 264. The shell inner threads 264 can be configured to engage the structure outer threads 262 (e.g., same pitch, approximate radius, etc.). The shell inner threads 264 can be configured to dig into and fix to the inner structure 258 if the inner structure 258 does not have structure outer threads 262 corresponding to the shell inner threads 264.

The outer shell 252 can have one or more outer shoulder 309 configurations on the inner surface of the outer shell wall 296. The outer shoulder 309 can be at the proximal end, distal end, center portion, or combinations thereof, of the outer shell 252. The outer shoulder 309 can be a tapered ramp surface. For example, when the inner structure 258 is forced into the outer shell 252, the outer shoulder 309 can mate and receive pressure from the inner shoulder 320. The mating of the outer shoulder 309 and the inner shoulder 320 can minimize or eliminate unbearable loads transferred between the shell inner thread 264 and the structure outer thread 262.

Figures 157a and 157b illustrate that the inner structure 258 can have structure outer threads 262. The structure outer threads 262 can be configured to engage corresponding shell inner threads 264 during use. The shell inner threads 264 can be configured to dig into and fix to the inner structure 258 if the inner structure 258 does not have structure outer threads 262 corresponding to the shell inner threads 264. The structure outer threads 262 and/or shell inner threads 264 can be machined (e.g., by lathing).

The inner structure 258 can have one or more inner shoulder 320 configurations on the outer surface of the inner structure 258. The inner shoulder 320 can be at the proximal end, distal end, center portion, or combinations thereof, of the inner structure 258. The inner shoulder 320 configuration can be a tapered ramp surface. The inner structure 258 can have an inner seam 318 between the inner shaft 300 and the inner shoulder 320.

The inner structure 258 can have an inner structure wall 302 surrounding the hollow length.

Figures 158a and 158b illustrate that outer structure can have rod attachment thread 308 in the outer shell distal tip 305. The rod attachment thread 308 can be configured to attach to a deployment rod.

The outer shell 252 can have an outer shell distal tip 305. The outer shell distal tip 305 can be sharpened to a point, sharpened to a flat, flattened, rounded (e.g., hemi-spherical, hemi-ovular), traumatic or atraumatic, or combinations thereof. The outer shell distal tip 305 can be configured to drive through soft tissue and/or hard (e.g., bone) tissue. The outer shell distal tip 305 can have an outer shell distal port 256, for example located at the radial center of the outer shell distal tip 305. A narrowed (or not narrowed) hollow length can pass through the outer shell distal tip 305. The hollow length, for example at the distal and/or proximal ends can have rod attachment thread 308.

The outer shell 252 and inner structure 258 can expand when heated and contract when cooled. The outer shell 252 and inner structure 258 can be more malleable when heated and less malleable when cooled. The outer shell 252 can be heated during use, for example, to ease entry of the internal structure into the hollow length. The outer shell 252 can then be cooled when the internal structure is satisfactorily entered into the outer shell 252. The inner structure 258 can be cooled during use, for example, to ease entry of the internal structure into the hollow length. The inner structure 258 can then be heated when the internal structure is satisfactorily entered into the outer shell 252.

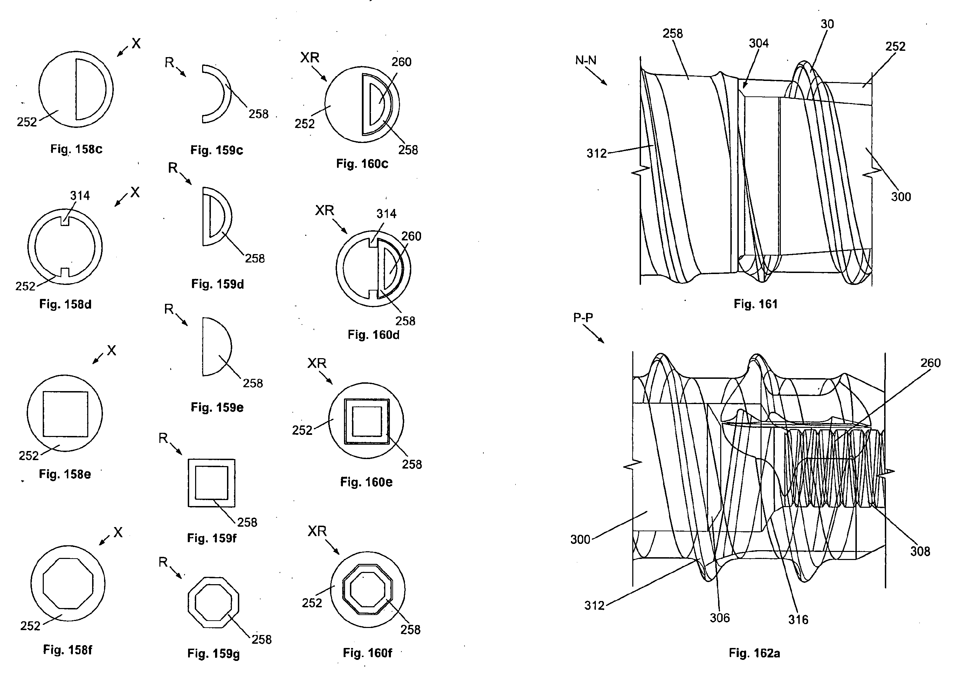

Figure 158c illustrates that the outer shell 252 can have a D-shaped hollow length. Figure 158d illustrates that the outer shell 252 can have one, two or more outer guides 314 protruding radially inward toward the hollow length. The outer guides 314 can be oppositely positioned to each other. The outer guides 314 can partially or completely transect the hollow length. Figure 158e illustrates that the outer shell 252 can have a hollow length with a square-shaped transverse cross-sectional configuration. Figure 158f illustrates that the outer shell 252 can have a hollow length with a hexagonal-shaped (or other polygonal-shaped) transverse cross-sectional configuration.

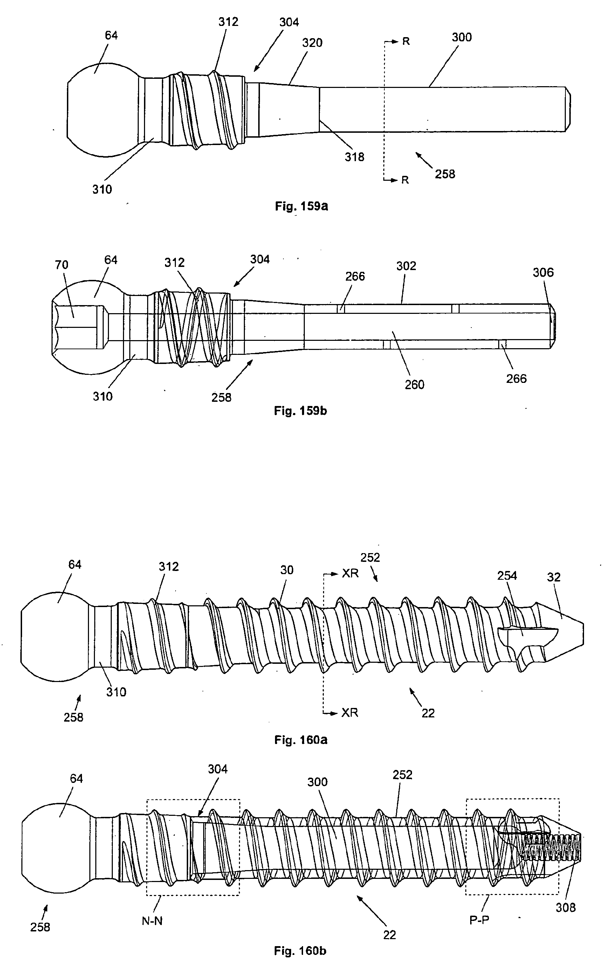

Figures 159a and 159b illustrate that the inner structure 258 can have an inner structure proximal stop 304. The inner structure proximal stop 304 can be radially raised from the radius of the inner shoulder 320 and/or inner shaft 300 directly adjacent to the inner structure proximal stop 304.

The inner structure 258 can have a structure external thread 312. The structure external thread 312 can be configured to engage the tissue at the target site (e.g., bone and/or soft tissue).

The inner structure 258 can have an inner neck 310 between the proximal end cap 64 and the structure external thread 312. The structure external thread 312 can be directly adjacent to the proximal end cap 64 and no inner neck 310 can be between the proximal end cap 64 and the structure external thread 312.

Figure 159c illustrates that the inner structure 258 can a C-shaped transverse cross-sectional configuration of the inner surface and/or the outer surface. Figure 159d illustrates that the inner structure 258 can have a hollow D-shaped transverse cross-sectional configuration of the inner surface and/or the outer surface. Figure 159e illustrates that the inner structure 258 can have a solid D-shaped transverse cross-sectional configuration of the inner surface and/or the outer surface with no hollow length. Figure 159f illustrates that the inner structure 258 can have a hollow square-shaped transverse cross-sectional configuration of the inner surface and/or the outer surface. Figure 159g illustrates that inner structure 258 can have a hexagonal-shaped (or other polygonal-shaped) transverse cross-sectional configuration of the inner surface and/or the outer surface.

Figures 160a and 160b illustrate that the structure external thread 312 can be at approximately the same radius as the outer shell thread 66 (the thread can be expandable thread 66 and/or unexpandable thread 30 depending on the variation desired). The structure external thread 312 can align with the outer shell thread 66. For example, the proximal terminal end of the outer shell 252 can be adjacent to the distal terminal end of the structure external thread 312.

During use, the structure external thread 312 and the outer shell thread 66 can engage tissue at the target site.

Figures 160c and 160d illustrate that an outer structure having a D-shaped transverse cross-sectional configuration of the hollow length (e.g., the outer shell 252 of Figure 158c) and/or having oppositely opposed outer guides 314 (e.g., the outer shell 252 of Figure 158d), can slidably receive an inner structure 258 having a C-shaped or D-shaped transverse cross-sectional configuration (e.g., the inner structures 258 of Figures 159c, 159d or 159e).

Figure 160d can have one or two or more separate sections of the hollow length, as shown in cross-section. The two or more separate sections of the hollow length can be not directly in fluid communication with each other. Different fillers can be delivered in each separate sections of the hollow length. For example, different components of a two-part epoxy can be delivered separately in each separate section of the hollow length. The epoxy components can mix in the treatment site. The inner structure 258 can rotationally interference fit against the outer guides 314.

Figure 160e illustrates that the inner structure 258 having a square-shaped transverse cross-sectional configuration of the hollow length (e.g., the outer shell 252 of Figure 158e) can slidably receive an inner structure 258 having a square-shaped transverse cross-sectional configuration of the outer surface (e.g., the inner structure 258 of Figure 159f). The transverse cross-section of the inner surface of the inner structure 258 can be hexagonal or circular.

Figure 160f illustrates that the inner structure 258 having a hexagonal-shaped (or other polygonal shaped) transverse cross-sectional configuration of the hollow length (e.g., the outer shell 252 of Figure 158f) can slidably receive an inner structure 258 having a hexagonal-shaped (or other polygonal shape matching the inner surface of the outer shell 252) transverse cross-sectional configuration of the outer surface (e.g., the inner structure 258 of Figure 159g). The transverse cross-section of the inner surface of the inner structure 258 can be hexagonal or circular.

The configurations shown in Figures 160c through 160f can transmit rotational torque about the longitudinal axis between the inner structure 258 and the outer shell 252. The sides of the hexagonal or square (or triangular, pentagonal, or other polygonal) transverse cross-sections, (e.g., of Figures 160f and 160e, respectively) can act as detents to interference fit in the rotational degree of freedom about the longitudinal axis (i.e., perpendicular with the plane shown of Figures 160e and 160f). The straight inner surface (as viewed in transverse cross-section) of the D-shaped inner surface of the outer shell 252, (e.g., of Figure 160c) and/or the outer guides 314 (e.g., of Figure 160d) can act as detents to interference fit in the rotational degree of freedom about the longitudinal axis (i.e., perpendicular with the plane shown of Figures 160c and 160d).

A torque can be applied to the inner structure 258 that can then be transferred through the inner structure 258 and the detent to the outer shell 252. A torque can be applied to the outer shell 252 that can then be transferred through the outer shell 252 and the detent to the inner structure 258.

Figure 161 illustrates that the inner structure proximal stop 304 can abut the proximal end of the outer shell 252. The outer shell 252 can interference fit against the inner structure proximal stop 304. The outer shell 252 surface adjacent to the inner structure 258 can mate with the surface of the inner structure 258. The inner structure proximal stop 304 can be configured so the adjacent inner structure 258 and outer shell 252 can have no substantial surface irregularity at the joint between the inner structure 258 and the outer shell 252.

Figures 162a illustrates that, in some variations, the internal structure distal tip 305 can not contact the distal shell inner stop 316. For example, the outer shell 252 can interference fit against the inner structural proximal stop. The device can limit expansion, for example, when the device is chamfered.

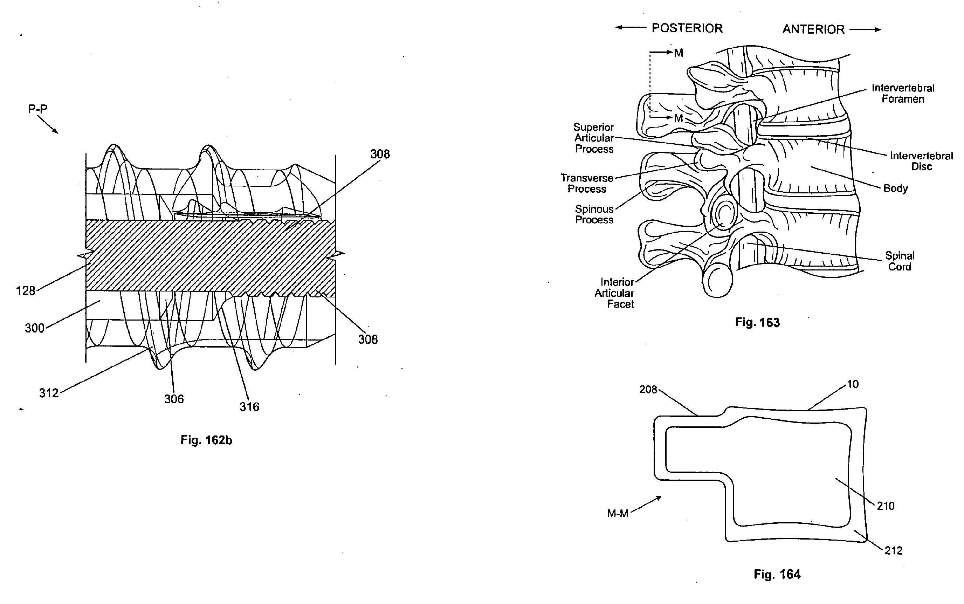

Figure 162b illustrates the device according to the invention having a deployment rod 128, for example to transmit the compressive force to the distal end of the outer shell 252. The deployment rod 128 can be inserted through the hollow length of the inner structural element. The deployment rod 128 can substantially fill the hollow length or leave a significant portion of the hollow length empty when deployed into the hollow length. The deployment rod 128 can be releasably attached to the rod attachment thread 308, for example via a deployment rod thread 49.

The deployment rod 128 can be detached from the outer shell 252 after the device is deployed. The deployment rod 128 can remain attached to the outer shell 252 after the device is deployed. The deployment rod 128 can be left in the device in the treatment site.

High strength materials can be used to make the inner structure 258 and/or outer shell 252.

Attachment elements can include the threads, sloped shoulders or other configurations used for attaching the inner structure 258 to the outer structure.

The outer shells 252 described in Figures 154a through 162b can be expandable and/or unexpandable. The outer shells 252 can have solid walls, walls with fenestrations, walls with struts and cells (e.g., an expandable scaffold, or stent-like configuration), other configurations shown herein for elements of the attachment device, or combinations thereof.

Figure 163 illustrates a side view of a spine 202. Figure 164 illustrates that harder, cortical bone 212 surrounds softer, cancellous bone 246 in the vertebra 10.

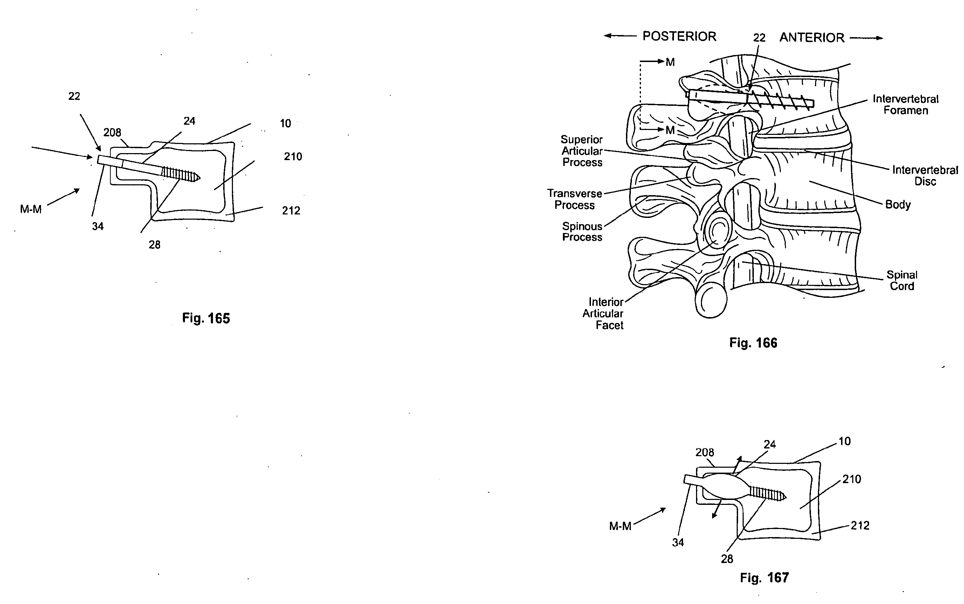

Figure 165 illustrates that the expandable attachment device 22 can be translated and/or rotated into the pedicle 208 and/or into the vertebral body 10. The expanded section 24 can be positioned in the cortical bone 212.

Figures 166 and 167 illustrate that the expandable section 24 can be radially expanded, for example in the cancellous bone 246 of the pedicle 208 and/or the vertebral body 10. The radius of the radially expanded section 24 can be larger than the entry hole created to insert the attachment device into the vertebra 10.

The proximal end 34 can extend from the bone 228. A separate device, such as a fixation rod 14 or plate 220, can be attached to the proximal end 34.

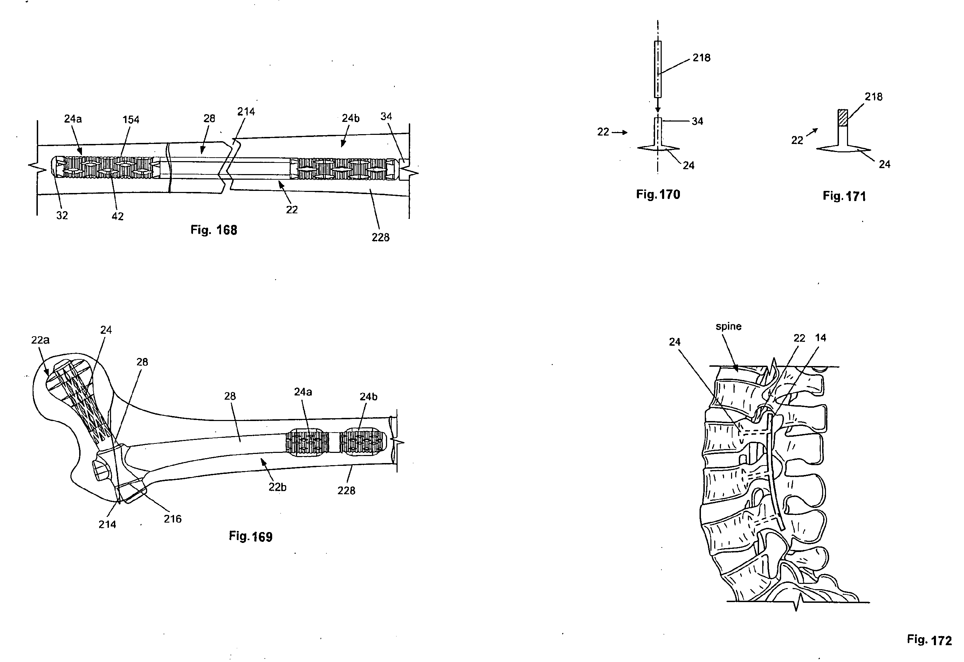

Figure 168 illustrates that an expandable attachment device 22 can be used to treat a long bone 228 break, such as in the femur or humerus. The expandable attachment device 22 can be inserted into the cancellous and/or cortical part of the bone 212. The expandable attachment device 22 can be positioned to have a first expandable section 24a on a first side of the bone fracture 214. The expandable attachment device 22 can be positioned to have a second expandable section 24b on a second side of the bone fracture 214. The expandable attachment device 22 can have an unexpandable section 28 between the first and second expandable sections 24a, 24b. The unexpandable section 28 can be positioned across the bone fracture 214.

Figure 169 illustrates that a first expandable attachment device 22a can be placed in a first section of the bone 228a (e.g., the femur head). A second expandable attachment device 22b can be placed in a second section of the bone 228b. The second expandable attachment device 22b can have a collar 216 configured to fixedly receive the unexpandable section 28 of the first expandable attachment device 22a. The unexpandable section 28 of the first expandable attachment device 22a can be fixedly attached to the collar 216.

Figures 170 and 171 illustrates that the expandable attachment device 22 can have an end attachment 218 configured to be attached, as shown by arrow, to the proximal end 34. For example, the expandable attachment device 22 can be positioned in a bone 228 and radially expanded. The end attachment 218 can be attached to the proximal end 34, as shown in Figure 173. The end attachment 218 can be configured to attach to a separate device, such as a fixation rod 14 or plate 220, as shown in Figure 172.

Figure 174 through 176 illustrates that the expandable attachment device 22 can be deployed by radially expanding the first expandable section 24a at a first end, and concurrently or subsequently, radially expanding the second expandable section 24b at a second end.

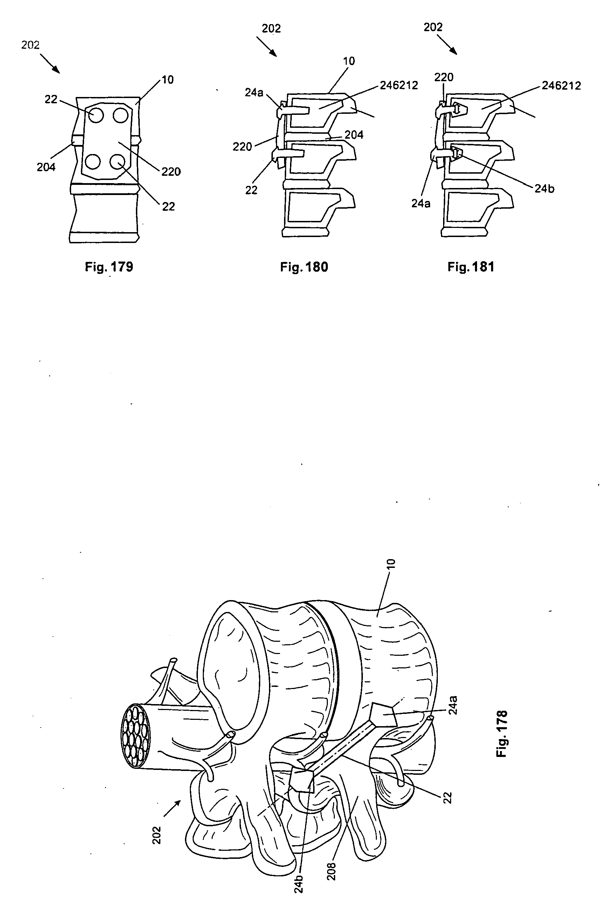

Figures 177 and 178 illustrate that the expandable attachment device 22 can be positioned so the first expandable section 24a can be radially expanded in the pedicle 208 or vertebral body 10. The second expandable section 24b can be radially expanded in the pedicle 208, vertebral body 10, or outside the bone 228, for example in the soft tissue or in a virtual space. A separate device, such as a fixation rod 14 or plate 220 can be attached to the second expandable section 24b.

Figures 179 through 181 illustrate that a fixation plate 220 can be attached to the anterior side of the spine 202. Figure 180 illustrates that the expandable attachment devices 22 can be attached to the fixation plate 220 and the first expandable section 24a can be radially expanded. Figure 181 illustrates that the second expandable sections 24b of the expandable attachment devices 22 can be positioned in the cancellous bone 246. The second expandable sections 24b can be radially expanded, as shown by arrows, in the cancellous bone 246, for example in the vertebral body 10.

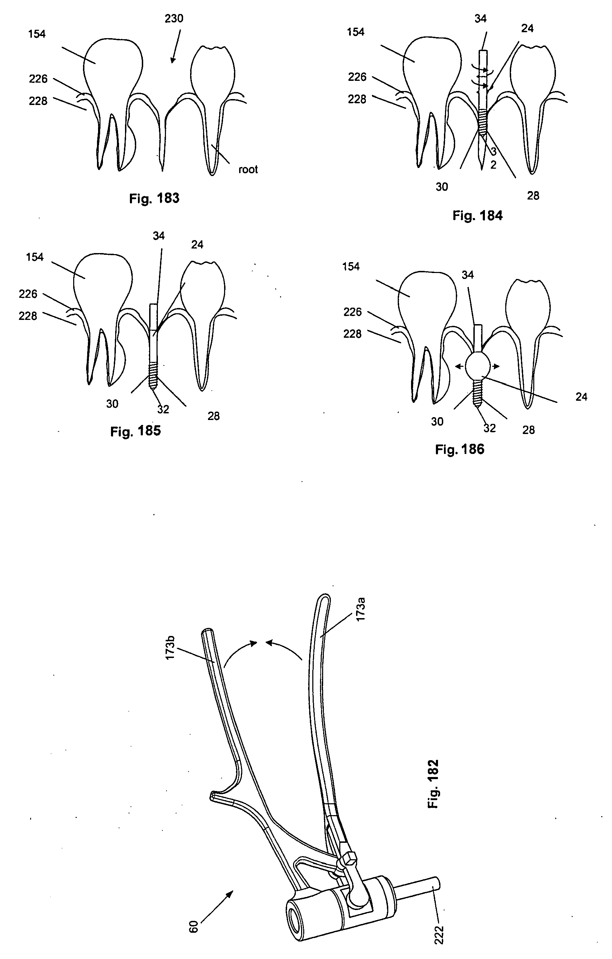

Figure 182 illustrates that the deployment tool 60 can have a first handle 224a rotatably attached to a second handle 224b. Rotating the first handle 224a and the second handle 224b towards each other, as shown by arrows, can result in longitudinal compression 44 of the expandable section 24 of the expandable attachment device 22. See the incorporated applications for additional elements of the deployment tool 60. The expandable attachment device 22 can be removably attached to the deployment head 222.

Figure 183 illustrates that an oral space can have a missing tooth 230. The missing tooth 230 can be surrounded on one side, both sides or neither side, by teeth 154. The gum 226, bone 228, and teeth roots 232 are also shown.

Figure 184 illustrates that the expandable attachment device 22 can be screwed (e.g., rotation and translation), as shown by arrows, into the missing tooth 230 space in the bone 228. The unexpandable thread 30 can compact or cut bone 228 as the expandable attachment device 22 is inserted into the missing tooth 230 space in the bone 228. Figure 185 illustrates that the expandable support device can be fully inserted into the bone 228. The proximal end 34 can extend above the gum 226.

Figure 186 illustrates that the expandable section 24 can be radially expanded, as shown by arrows, for example with the expandable support device fully inserted into the bone 228.

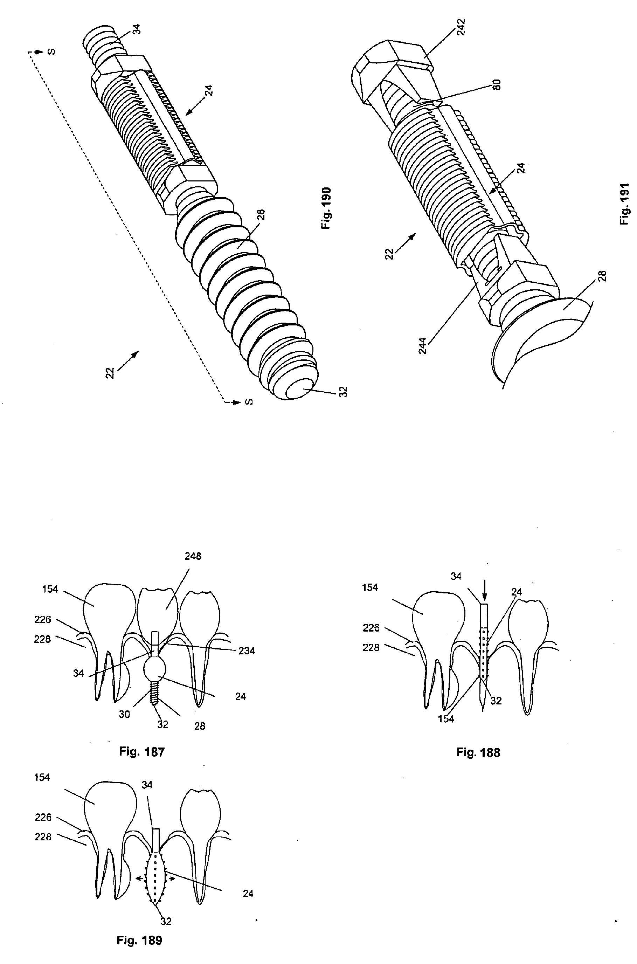

Figure 187 illustrates that a replacement tooth 248 can be fixedly or removably attached to the proximal end 34. The proximal end 34 can be configured to attach to the replacement tooth 248 (e.g., thread, one or more latches, clasps, locks). The replacement tooth 248 can be positioned between the adjacent teeth 154. The space between the replacement tooth 248 and the gum 226 can be partially or completely filled by a filler 234, for example a biocompatible cement (e.g., a bone cement).

Figure 188 illustrates that the expandable attachment device 22 can have unidirectional and/or one-way teeth 154 along all or part of the length of the expandable section 24. The expandable section 24 can be along substantially the entire length of the expandable attachment device 22, for example, except for the proximal end 34 configured to attach to the replacement tooth 248.

Figure 189 illustrates that the expandable section 24 can be radially expanded, as shown by arrows. The replacement tooth 248 can then be attached as shown in Figure 178.

Figures 190 and 191 illustrate that the expandable section 24 can have expandable threads 66 around one or more sections of the expandable section 24(e.g., for example on opposite sides of the expandable section 24, as shown). The distal wedge 242 and/or the proximal wedge 244 can have threads 50 on the internal diameter or be threadless on the internal diameter. The internal threads 250 can engage the proximal length of the center shaft 80 (e.g., the proximal length of the center shaft 80 have a smaller, larger or the same diameter as compared to the diameter of the distal length of the center shaft 80). The proximal wedge 244 can have an internal diameter that can be larger than the threads 50 on the center shaft 80 so the proximal wedge 244 can slide freely over the distal length of the center shaft 80 and/or the proximal length of the center shaft 80.

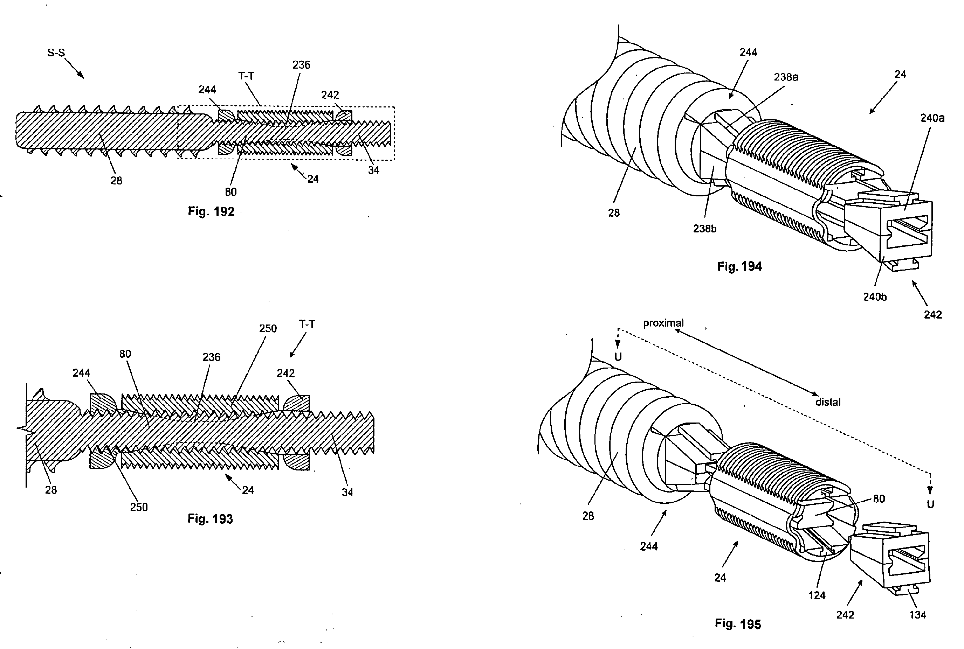

Figures 192 and 193 illustrate that the outer diameter of the unexpandable section 28 can be substantially equivalent to the outer diameters of the expandable section 24 (e.g., in a radially contracted configuration) and/or the wedges 130. The outer diameter of the expandable section 24 (e.g., in a radially contracted configuration) can be slightly larger than, smaller than, or substantially equivalent to the outer diameter of the unexpandable section 28.

The internal diameter of the expandable section 24 and the internal diameter of one or more of the wedges 130 (e.g., shown as only the proximal wedge 244 in Figures 192 and 193) can have internal threads 250 and/or teeth 154, for example, configured to engage threads 50 and/or teeth 154 on the center shaft 80.

The center shaft 80 can have a reduced diameter (as shown) at a length near the longitudinal middle of the center shaft 80. The internal threads 250 or teeth 154 (e.g., on the inner diameter of the expandable section 24) might not engage the center shaft 80 along the length having the reduced diameter, for example because of no geometric overlap and/or the absence of teeth 154 or threads 50 along the outer diameter of the center shaft 80 along the length having the reduced diameter.

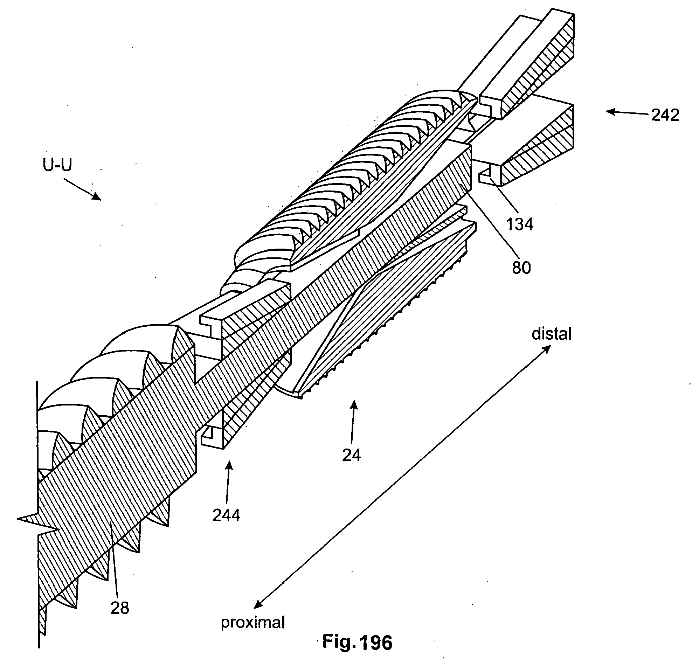

Figures 194, 195 and 196 illustrate that the wedges 130 can be segmented. For example, the proximal wedge 244 can have adjacent and/or attached proximal wedge 244 first and second segments. The distal wedge 242 can have adjacent and/or attached distal wedge 242 first and second segments.

The wedge 130 segments can be configured to individually or jointedly fixedly (e.g., via ratcheting on the center shaft 80 and/or wedge 130) or releasably attach to the center shaft 80 and/or expandable section 24. For example, the expandable section 24 and/or center shaft 80 can have one or more male or female configurations (e.g., guide slots 124, such as T-slots, as shown) and the wedge 130 segment can have one or more corresponding female or male segments (e.g., wedge rails 134, such as T-extensions, as shown). When the proximal wedge 244 is forced distally and/or the distal wedge 242 is forced proximally, one or both wedges 130 can force 132 the expandable section 24 to radially expand. When the proximal wedge 244 is forced proximally and/or the distal wedge 242 is forced 132 distally, one or both wedges 130 can force the expandable section 24 to radially contract.

Any or all elements of the expandable attachment device 22 and/or other devices or apparatuses described herein can be made from, for example, a single or multiple stainless steel alloys, nickel titanium alloys (e.g., Nitinol), cobalt-chrome alloys (e.g., ELGILOY® from Elgin Specialty Metals, Elgin, IL; CONICHROME® from Carpenter Metals Corp., Wyomissing, PA), nickel-cobalt alloys (e.g., MP35N® from Magellan Industrial Trading Company, Inc., Westport, CT), molybdenum alloys (e.g., molybdenum TZM alloy, for example as disclosed in International Pub. No. WO 03/082363 A2, published 9 October 2003), tungsten-rhenium alloys, for example, as disclosed in International Pub. No. WO 03/082363, polymers such as polyethylene teraphathalate (PET), polyester (e.g., DACRON® from E. I. Du Pont de Nemours and Company, Wilmington, DE), poly ester amide (PEA), polypropylene, aromatic polyesters, such as liquid crystal polymers (e.g., Vectran, from Kuraray Co., Ltd., Tokyo, Japan), ultra high molecular weight polyethylene (i.e., extended chain, high-modulus or high-performance polyethylene) fiber and/or yarn (e.g., SPECTRA® Fiber and SPECTRA® Guard, from Honeywell International, Inc., Morris Township, NJ, or DYNEEMA® from Royal DSM N.V., Heerlen, the Netherlands), polytetrafluoroethylene (PTFE), expanded PTFE (ePTFE), polyether ketone (PEK), polyether ether ketone (PEEK), poly ether ketone ketone (PEKK) (also poly aryl ether ketone ketone), nylon, polyether-block copolyamide polymers (e.g., PEBAX® from ATOFINA, Paris, France), aliphatic polyether polyurethanes (e.g., TECOFLEX® from Thermedics Polymer Products, Wilmington, MA), polyvinyl chloride (PVC), polyurethane, thermoplastic, fluorinated ethylene propylene (FEP), absorbable or resorbable polymers such as polyglycolic acid (PGA), poly-L-glycolic acid (PLGA), polylactic acid (PLA), poly-L-lactic acid (PLLA), polycaprolactone (PCL), polyethyl acrylate (PEA), polydioxanone (PDS), and pseudo-polyamino tyrosine-based acids, extruded collagen, silicone, zinc, echogenic, radioactive, radiopaque materials, a biomaterial (e.g., cadaver tissue, collagen, allograft, autograft, xenograft, bone cement, morselized bone, osteogenic powder, beads of bone) any of the other materials listed herein or combinations thereof. Examples of radiopaque materials are barium sulfate, zinc oxide, titanium, stainless steel, nickel-titanium alloys, tantalum and gold.

Any or all elements of the expandable attachment device 22 and/or other devices or apparatuses described herein, can be, have, and/or be completely or partially coated with agents and/or a matrix a matrix for cell ingrowth or used with a fabric, for example a covering (not shown) that acts as a matrix for cell ingrowth. The matrix and/or fabric can be, for example, polyester (e.g., DACRON® from E. I. Du Pont de Nemours and Company, Wilmington, DE), poly ester amide (PEA), polypropylene, PTFE, ePTFE, nylon, extruded collagen, silicone, any other material disclosed herein, or combinations thereof.

The expandable attachment device 22 and/or elements of the expandable attachment device 22 and/or other devices or apparatuses described herein and/or the fabric can be filled, coated, layered and/or otherwise made with and/or from cements, fillers, glues, and/or an agent delivery matrix known to one having ordinary skill in the art and/or a therapeutic and/or diagnostic agent. Any of these cements and/or fillers and/or glues can be osteogenic and osteoinductive growth factors.

Examples of such cements and/or fillers 234 includes bone 228 chips, demineralized bone matrix (DBM), calcium sulfate, coralline hydroxyapatite, biocoral, tricalcium phosphate, calcium phosphate, polymethyl methacrylate (PMMA), biodegradable ceramics, bioactive glasses, hyaluronic acid, lactoferrin, bone morphogenic proteins (BMPs) such as recombinant human bone morphogenetic proteins (rhBMPs), other materials described herein, or combinations thereof.

The agents within these matrices can include any agent disclosed herein or combinations thereof, including radioactive materials; radiopaque materials; cytogenic agents; cytotoxic agents; cytostatic agents; thrombogenic agents, for example polyurethane, cellulose acetate polymer mixed with bismuth trioxide, and ethylene vinyl alcohol; lubricious, hydrophilic materials; phosphor cholene; antiinflammatory agents, for example non-steroidal anti-inflammatories (NSAIDs) such as cyclooxygenase-1 (COX-1) inhibitors (e.g., acetylsalicylic acid, for example ASPIRIN® from Bayer AG, Leverkusen, Germany; ibuprofen, for example ADVIL® from Wyeth, Collegeville, PA; indomethacin; mefenamic acid), COX-2 inhibitors (e.g., VIOXX® from Merck & Co., Inc., Whitehouse Station, NJ; CELEBREX® from Pharmacia Corp., Peapack, NJ; COX-1 inhibitors); immunosuppressive agents, for example Sirolimus (RAPAMUNE®, from Wyeth, Collegeville, PA), or matrix metalloproteinase (MMP) inhibitors (e.g., tetracycline and tetracycline derivatives) that act early within the pathways of an inflammatory response. Examples of other agents are provided in Walton et al, Inhibition of Prostoglandin E2 Synthesis in Abdominal Aortic Aneurysms, Circulation, July 6, 1999, 48-54; Tambiah et al, Provocation of Experimental Aortic Inflammation Mediators and Chlamydia Pneumoniae, Brit. J. Surgery 88 (7), 935-940; Franklin et al, Uptake of Tetracycline by Aortic Aneurysm Wall and Its Effect on Inflammation and Proteolysis, Brit. J. Surgery 86 (6), 771-775; Xu et al, Spl Increases Expression of Cyclooxygenase-2 in Hypoxic Vascular Endothelium, J. Biological Chemistry 275 (32) 24583-24589; and Pyo et al, Targeted Gene Disruption of Matrix Metalloproteinase-9 (Gelatinase B) Suppresses Development of Experimental Abdominal Aortic Aneurysms, J. Clinical Investigation 105 (11), 1641-1649.

Other examples of fractures types that can be treated with the disclosed device and method include Greenstick fractures, transverse fractures, fractures across growth plates, simple fractures, wedge fractures, complex fractures, compound fractures, complete fractures, incomplete fractures, linear fractures, spiral fractures, transverse fractures, oblique fractures, comminuted fractures, impacted fractures, and soft tissue tears, separations (e.g., avulsion fracture), sprains, and combinations thereof. Plastic deformations of bones can also be treated with the disclosed device and method.

Other examples of bones that can be treated with the disclosed device and method include the fingers (e.g., phalanges), hands (e.g., metacarpals, carpus), toes (e.g., tarsals), feet (metatarsals, tarsus), legs(e.g., femur, tibia, fibula), arms (e.g., humerus, radius, ulna), scapula, coccyx, pelvis, clavicle, scapula, patella, sternum, ribs, or combinations thereof.

Devices, elements and configurations disclosed as expandable support devices in the following publicatons can be used for the expandable section in the present application: WO2006034436 A2, WO 2006116760 A2, WO 2006037013 A1, WO 2006068682, WO 2006042334 A2, WO 2007076374 A2, WO 2007041665 A2, WO 2007009107 A2, WO 2007076308 A2, WO 2007076377 A2, WO 2007084239 A2, US 2007-0032791 A1.

All dimensions shown herein are exemplary. The dimensions shown herein can at least be expanded to ranges from about 50% to about 150% of the exemplary dimension shown herein, more narrowly from about 75% to about 125% of the exemplary dimension shown herein.

The use of the term "radial expansion" herein refers to both a volumetric increase of an element, or an increase in the radial dimension of the element itself, or the increase in the maximum radius of the element as measured from the expandable attachment device 22 axis.

Any elements described herein as singular can be pluralized (i.e., anything described as "one" can be more than one). Any species element of a genus element can have the characteristics or elements of any other species element of that genus.

## Claims

1. An attachment device (22) for deployment to biological tissue comprising:
an expandable outer shell (252), wherein the outer shell has a hollow length (260), and wherein the outer shell comprises a first attachment element on the radial inside of the outer shell;
an inner structure (258), wherein the inner structure comprises a second attachment element on the radial outside of the inner structure; and
wherein the second attachment element is configured to attach to the first attachment element;
**characterised in that** the inner structure comprises a hollow shaft (300) and **in that** the device further comprises a deployment rod (128) that is configured to be inserted through the hollow shaft and releasably attached to a distal end of the outer shell, via a rod attachment thread (308) in the outer shell, to allow a translation of the distal end of the outer shell relative to the inner structure thereby to apply a compressive force to the outer shell to expand the outer shell from a radially contracted state to a radially expanded state.

2. The device of Claim 1, wherein the outer shell comprises a helical thread on the outside of the outer shell.

3. The device of Claim 1 or Claim 2, wherein the inner structure is press-fit to the outer shell.

4. The device of claim 3, wherein the press-fit includes an inner shoulder (320) of the inner structure engaging an outer shoulder (309) of the outer shell.

5. The device of claim 1, the attachment device having a longitudinal axis and further comprising an anti-torque element configured to minimize rotation of the inner structure relative to the outer shell with respect to the longitudinal axis.

6. The device of Claim 5, wherein the anti-torque element comprises a locking pin (273).

7. The device of Claim 6, wherein the locking pin crosses the outer shell wall and the inner structure wall.

8. The device of Claim 5, wherein the anti-torque element comprises a thread (264) on the inner surface of the outer shell.

9. The device of Claim 8, wherein the anti-torque element comprises a thread (262) on the outer surface of the inner structure.

10. The device of Claim 5, wherein the anti-torque element comprises a detent on the inner surface of the outer shell.

11. The device of claim 10, wherein the anti-torque element further comprises a hexagonal transverse cross-sectional configuration of the outer surface of the inner structure engaging the detent on the inner surface of the outer shell.

12. The device of any one of the preceding claims, wherein the deployment rod is releasably attached to a distal end (305) of the outer shell via a rod attachment thread (308).

13. The device of any one of the preceding claims, wherein the inner structure is fixedly attached to the outer shell.

14. The device of any one of the preceding claims, further comprising filler to be delivered to a target site through the inner structure and the outer shell.

15. The device of any one of the preceding claims, wherein the deployment rod is constructed and arranged so that, upon being pulled proximally, it will expand the outer shell.

## Patentansprüche

1. Befestigungsvorrichtung (22) für einen Einsatz an biologischem Gewebe, umfassend:
eine ausdehnbare äußere Hülle (252), wobei die äußere Hülle eine hohle Länge (260) aufweist, und wobei die äußere Hülle ein erstes Befestigungselement an der radialen Innenseite der äußeren Hülle umfasst;
eine innere Struktur (258), wobei die innere Struktur ein zweites Befestigungselement an der radialen Außenseite der inneren Struktur umfasst;
und
wobei das zweite Befestigungselement ausgestaltet ist, an dem ersten Befestigungselement angebracht zu sein;
**dadurch gekennzeichnet, dass** die innere Struktur einen hohlen Schaft (300) umfasst und dass die Vorrichtung ferner umfasst
einen Einsatzstab (128), welcher ausgestaltet ist, durch den hohlen Schaft eingesetzt zu werden und lösbar an einem distalen Ende der äußeren Hülle über ein Stabbefestigungsgewinde (308) in der äußeren Hülle angebracht zu werden, um eine Verschiebung des distalen Endes der äußeren Hülle relativ zu der inneren Struktur zu ermöglichen, um dadurch eine zusammenpressende Kraft auf die äußere Hülle aufzubringen, um die äußere Hülle aus einem radial zusammengezogenen Zustand in einen radial ausgedehnten Zustand auszudehnen.

2. Vorrichtung nach Anspruch 1, wobei die äußere Hülle ein spiralförmiges Gewinde an der Außenseite der äußeren Hülle umfasst.

3. Vorrichtung nach Anspruch 1 oder Anspruch 2, wobei die innere Struktur in die äußere Hülle eingepresst ist.

4. Vorrichtung nach Anspruch 3, wobei die Presspassung eine innere Schulter (320) der inneren Struktur aufweist, welche sich in Eingriff mit einer äußeren Schulter (309) der äußeren Hülle befindet.

5. Vorrichtung nach Anspruch 1, wobei die Befestigungsvorrichtung eine Längsachse aufweist und ferner ein Antidrehmomentelement umfasst, welches ausgestaltet ist, eine Drehung der inneren Struktur relativ zu der äußeren Hülle bezogen auf die Längsachse zu minimieren.

6. Vorrichtung nach Anspruch 5, wobei das Antidrehmomentelement einen Verriegelungsstift (273) umfasst.

7. Vorrichtung nach Anspruch 6, wobei der Verriegelungsstift die äußere Hüllenwand und die innere Strukturwand kreuzt.

8. Vorrichtung nach Anspruch 5, wobei das Antidrehmomentelement ein Gewinde (264) an der inneren Fläche der äußeren Hülle umfasst.

9. Vorrichtung nach Anspruch 8, wobei das Antidrehmomentelement ein Gewinde (262) an der äußeren Fläche der inneren Struktur umfasst.

10. Vorrichtung nach Anspruch 5, wobei das Antidrehmomentelement eine Arretierung an der inneren Fläche der äußeren Hülle umfasst.

11. Vorrichtung nach Anspruch 10, wobei das Antidrehmomentelement ferner eine hexagonal quer verlaufende Querschnittskonfiguration der äußeren Fläche der inneren Struktur umfasst, welche sich in Eingriff mit der Arretierung an der inneren Fläche der äußeren Hülle befindet.

12. Vorrichtung nach einem der vorhergehenden Ansprüche, wobei der Einsatzstab lösbar an einem distalen Ende (305) der äußeren Hülle über ein Stabbefestigungsgewinde (308) angebracht ist.

13. Vorrichtung nach einem der vorhergehenden Ansprüche, wobei die innere Struktur fest an der äußeren Hülle angebracht ist.

14. Vorrichtung nach einem der vorhergehenden Ansprüche, ferner umfassend ein Füllmaterial, welches einem Zielort durch die innere Struktur und die äußere Hülle zuzuführen ist.

15. Vorrichtung nach einem der vorhergehenden Ansprüche, wobei der Einsatzstab derart konstruiert und angeordnet ist, dass er die äußere Hülle ausdehnen wird, wenn er proximal gezogen wird.

## Revendications

1. Dispositif de fixation (22) destiné au déploiement jusqu'à des tissus biologiques, comprenant :
une coque extérieure expansible (252), où la coque extérieure présente une longueur creuse (260), et où la coque extérieure comporte un premier élément de fixation sur la face radiale interne de la coque extérieure ;
une structure intérieure (258), où la structure intérieure comporte un deuxième élément de fixation sur la face radiale externe de la structure interne ;
et
où le deuxième élément de fixation est configuré pour être fixé au premier élément de fixation ;
**caractérisé en ce que** la structure intérieure comprend un arbre creux (300) et **en ce que** le dispositif comprend par ailleurs
une tige de déploiement (128) qui est configurée pour être insérée à travers l'arbre creux et est fixée de manière amovible à une extrémité distale de la coque extérieure, par l'intermédiaire d'un filetage (308) de fixation de tige dans la coque extérieure, pour permettre une translation de l'extrémité distale de la coque extérieure par rapport à la structure intérieure, afin d'appliquer ainsi une force de compression à la coque extérieure, en vue de dilater la coque extérieure pour la faire passer d'un état radialement rétracté à un état radialement déployé.

2. Dispositif selon la revendication 1, dans lequel la coque extérieure comprend un filetage hélicoïdal à l'extérieur de la coque extérieure.

3. Dispositif selon la revendication 1 ou 2, dans lequel la structure intérieure est emmanchée à force avec la coque extérieure.

4. Dispositif selon la revendication 3, dans lequel l'emmanchement à force inclut le fait qu'un épaulement intérieur (320) de la structure intérieure entre en prise avec un épaulement extérieur (309) de la coque extérieure.

5. Dispositif selon la revendication 1, le dispositif de fixation présentant un axe longitudinal et comprenant en outre un élément anticouple configuré pour réduire à un minimum la rotation entre la structure intérieure et à la coque extérieure, par rapport à l'axe longitudinal.

6. Dispositif selon la revendication 5, dans lequel l'élément anticouple comprend une goupille de verrouillage (273).

7. Dispositif selon la revendication 6, dans lequel la goupille de verrouillage traverse la paroi de la coque extérieure et la paroi de la structure intérieure.

8. Dispositif selon la revendication 5, dans lequel l'élément anticouple comporte un filetage (264) sur la surface interne de la coque extérieure.

9. Dispositif selon la revendication 8, dans lequel l'élément anticouple comprend un filetage (262) sur la surface externe de la structure intérieure.

10. Dispositif selon la revendication 5, dans lequel l'élément anticouple comprend un cran d'arrêt sur la surface interne de la coque extérieure.

11. Dispositif selon la revendication 10, dans lequel l'élément anticouple présente en outre une configuration à section transversale hexagonale de la surface externe de la structure intérieure, qui est en prise avec le cran d'arrêt sur la surface interne de la coque extérieure.

12. Dispositif selon l'une quelconque des revendications précédentes, dans lequel la tige de déploiement est fixée de manière amovible à une extrémité distale (305) de la coque extérieure, par l'intermédiaire d'un filetage (308) de fixation de tige.

13. Dispositif selon l'une quelconque des revendications précédentes, dans lequel la structure intérieure est liée de manière fixe à la coque extérieure.

14. Dispositif selon l'une quelconque des revendications précédentes, comprenant en outre une matière de remplissage devant être distribuée à un emplacement cible à travers la structure intérieure et la coque extérieure.

15. Dispositif selon l'une quelconque des revendications précédentes, dans lequel la tige de déploiement est conçue et agencée de manière à ce que, après avoir été tirée de façon proximale, elle déploie la coque extérieure.
